# EUROPÄISCHE PATENTSCHRIFT

(11) **EP 3 750 510 B1**
(45) Veröffentlichungstag und Bekanntmachung des Hinweises auf die Patenterteilung: **10.01.2024**
(21) Anmeldenummer: 20176847.0
(22) Anmeldetag: 27.05.2020
(51) Int. Cl.: A61F 2/28, A61B 17/70, A61M 27/00

(54) **MEDIZINISCHES IMPLANTAT ZUM GASAUSTAUSCH**
MEDICAL IMPLANT FOR GAS EXCHANGE
IMPLANT MÉDICAL DESTINÉ À L'ÉCHANGE DE GAZ

(30) Priorität: 12.06.2019 DE 102019115933
(43) Veröffentlichungstag der Anmeldung: 16.12.2020
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(56) Entgegenhaltungen:
- WO-A2-2007/106591
- US-A- 5 219 326
- US-A1- 2001 004 710
- KATSUJI MATSUNAGA ET AL: "Gas Permeability of Thermoplastic Polyurethane Elastomers", POLYMER JOURNAL, Bd. 37, Nr. 6, 15. Juni 2005 (2005-06-15), Seiten 413-417, XP055379460, JP ISSN: 0032-3896, DOI: 10.1295/polymj.37.413

## Beschreibung

Die Erfindung betrifft ein medizinisches Implantat zur Behandlung von Knochendefekten sowie ein Knochendefektbehandlungssystem aufweisend ein solches Implantat. Die Erfindung betrifft auch ein Verfahren zum Begasen einer Oberfläche eines medizinischen Implantats. Gegenstand der Erfindung ist somit ein medizinisches Implantat zur temporären Implantation in Knochenkavitäten.

Knochendefekte können beim Menschen vielfältige Ursachen haben. Häufige Ursachen sind Traumata und Infektionen. Knochendefekte heilen spontan nicht aus, wenn sie eine kritische Größe überschreiten. Es liegt dann ein sogenannter "critical-size-defect" vor. Zur Behandlung von Knochendefekten werden Knochenersatzmaterialien unterschiedlichster Struktur und Zusammensetzung, sowie allogenes Knochengewebe und autologes Knochengewebe klinisch eingesetzt.

Knochenersatzmaterialien sind seit Jahrzehnten bekannt und können aus unterschiedlichsten Materialien gefertigt sein. Typische anorganische Knochenersatzmaterialien sind Calciumsulfat (H. Dreesmann: Über Knochenplombierung. Klinische Chirurgie (1892) 804-810), Carbonatapatit (M. V. Vallet-Regi, J. M. Gonzalez-Cabbet: Calciumphosphates as substitution of bone tissues. Progress in Solid State Chemistry 32 (1-2) (2004)1-31), Hydroxylapatit, β-Tricalciumphosphat (R.W. Bucholz, A. Carlton, R. E. Holmes: Hydroxyapatite and tricalciumphosphate bone graft substitutes. The Orthopedic Clinics of North America 18(2) (1987) 323-334), Biogläser (H. Oonishi et al.: Particulate bioglass compared with hydroxyapatite as bone graft substitute. Clinical Orthopaedics and Related Research 334(1997) 316-325) und demineralisierte Knochenmatrix (M. E. Bolander, G. Balian: The use of demineralized bone matrix in the repair of segmental defects. Augmentation with extracted matrix proteins and comparison with autologous grafts. The Journal of bone and Joint Surgery Amercian Volume 68(8) 1986) 1264-1274). Daneben wurden auch Knochenersatzwerkstoffe auf organischer Basis, wie zum Beispiel Polyester, und auch Kombinationen aus anorganischen und organischen Werkstoffen für die Herstellung von Knochenersatzmaterialein eingesetzt (S. Higashi et al.: Polymer-hydroxyapatite composites for biodegradable bone fillers. Biomaterials 7(3) (1986) 183-187)

Die US 2001/0004710 A1 offenbart einen Platzhalter zum Behandeln einer Kavität eines Patienten, bei dem ein Ballon aufgeblasen werden kann und in den Ballon ein härtbares Biomaterial eingespeist werden kann. Ein Gasaustausch ist nicht vorgesehen. In dem Artikel "Gas Permeability of Thermoplastic Polyurethane Elastomers" von Katuji Matsunaga et a. in Polymer Jounal Vol. 37, No. 6, Seiten 413-417 (2205) ist ein Kunststoff bekannt, der für Sauerstoff und CO₂ durchlässig ist.

Im Dentalbereich werden Knochenersatzmaterialien mit Erfolg bei relativ kleinvolumigen Knochendefekten eingesetzt. Bei größeren Knochendefekten im Bereich der Extremitäten wird sehr häufig klinisch beobachtet, dass auch bei Verwendung von porösen Knochenersatzmaterialien das Knochengewebe nur oberflächlich in das Knochenersatzmaterial einwächst. Ähnliche Probleme treten bei der Transplantation von autologem Knochengewebe und auch bei Kombinationen von autologem Knochenmaterial und anorganischen Knochenersatzmaterialien auf. Das autologe Gewebe, das sich am weitesten vom gut durchbluteten Knochengewebe entfernt befindet, ist häufig geschädigt und stirbt vielfach ab.

Die der vorliegenden Erfindung zugrundeliegende Aufgabe kann also darin gesehen werden, eine Möglichkeit zu finden und gegebenenfalls ein medizinisches Implantat bereitzustellen, mit dem die Heilungschancen verbessert werden. Die Stabilisation und die Ossifikation des Knochendefekts sollen so schnell wie möglich und so unkompliziert wie möglich erreicht werden. Dabei können auch weitere Hilfsmittel eingesetzt, die den Heilungsprozess positiv beeinflussen.

Es wurde im Rahmen der vorliegenden Erfindung gefunden, dass ein wesentlicher Grund für die Beobachtung, dass autologes Gewebe, das sich am weitesten vom gut durchbluteten Knochengewebe entfernt befindet, häufig geschädigt ist und vielfach abstirbt, sehr wahrscheinlich darin begründet ist, dass diese Bereiche nicht mehr ausreichend mit Sauerstoff versorgt werden können und dass ein Abtransport des beim Stoffwechsel entstehenden Kohlendioxids und weiterer Stoffwechselprodukte nur erschwert möglich ist, weil keine Blutgefäße im Inneren des Knochenersatzmaterials vorhanden sind, bei denen das strömende Blut den Sauerstoff-Transport und den Abtransport des gebildeten Kohlendioxids gewährleistet.

Die Aufgabe der Erfindung besteht somit in der Entwicklung eines temporären medizinischen Implantats, das einen Gasaustausch mit seiner Umgebung ermöglichen soll. Das Implantat soll in der Lage sein, Sauerstoff an seiner Oberfläche an die Umgebung abzugeben und Kohlendioxid aus der Umgebung aufzunehmen und abzutransportieren. Die Abgabe des Sauerstoffs und die Aufnahme sollen kontinuierlich oder auch diskontinuierlich möglich sein. Der Gasaustausch und der Gastransport sollen durch ein Fluid erreicht werden, welches durch das medizinische Implantat strömen soll. Das medizinische Implantat soll explantierbar sein und soll nicht mit humanem oder tierischem Gewebe verwachsen können. Das temporäre medizinische Implantat soll einen Gasaustausch mit umgebenden Knochenersatzmaterialien und insbesondere mit autologem Knochengewebe und auch mit Zellen, wie Osteoblasten, besiedelten Knochenersatzmaterialien ermöglichen. Dadurch sollen die Zellen, insbesondere die Osteoblasten, nach ihrer Implantation in einen größeren Knochendefekt so lange am Leben erhalten werden, bis eine Versorgung der Zellen mit Sauerstoff durch neu gebildete Blutgefäße möglich ist. Sobald diese vom Organismus ausgebildet sind, soll das temporäre Implantat entfernt werden können.

Eine weitere Aufgabe der Erfindung ist die Entwicklung eines Knochenersatzmaterialsystems, das das zu entwickelnde temporäre medizinische Implantat als Bestandteil enthält.

Eine zusätzliche Aufgabe der vorliegenden Erfindung ist es auch, ein nicht medizinisches Verfahren zu entwickeln, mit dem Sauerstoff in einen Hohlraum abgegeben werden kann und gleichzeitig Kohlendioxid aus dem Hohlraum aufgenommen werden kann. Das Verfahren soll mit dem erfindungsgemäßen medizinischen Implantat durchführbar sein und in Hohlräumen angewendet werden, die kein Teil eines menschlichen oder tierischen Körpers sind.

Das medizinische Implantat ist neben seiner Platzhaltungsfunktion auch zum Gasaustausch mit dem umliegenden Gewebe bestimmt, wobei Sauerstoff oder ein Sauerstoff enthaltendes Spülgasgemisch oder eine mit Sauerstoff angereicherte Spülflüssigkeit als Fluid den Innenraum des Abstandhalters kontinuierlich oder diskontinuierlich durchströmt und über die permeable Außenwand des Implantats dem Gewebe Sauerstoff zugeführt und gleichzeitig Kohlendioxid abgeführt werden kann.

Die Aufgaben der Erfindung werden gelöst durch ein medizinisches Implantat zur Behandlung von Knochendefekten aufweisend mindestens einen Hohlkörper, der einen Innenraum im Inneren des Hohlkörpers begrenzt, eine Fluidzuleitung, die mit dem Innenraum des Hohlkörpers fluiddurchlässig verbunden ist, eine Fluidableitung, die mit dem Innenraum des Hohlkörpers fluiddurchlässig verbunden ist, wobei der Hohlkörper zumindest bereichsweise oder vollständig aus zumindest einem Kunststoff besteht, wobei der zumindest eine Kunststoff für Flüssigkeiten undurchlässig ist und für Sauerstoff und für Kohlendioxid durchlässig ist, so dass Sauerstoff aus einem durch den Hohlkörper geleiteten Fluid an die Umgebung des Hohlkörpers abgebbar ist und Kohlendioxid aus der Umgebung des Hohlkörpers in das Fluid aufnehmbar ist, wobei der Hohlkörper oder der zumindest eine Kunststoff des Hohlkörpers einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweist.

Bevorzugt ist das medizinische Implantat ein temporäres medizinisches Implantat.

Das durch den Hohlkörper geleitete Fluid muss zur Abgabe von Sauerstoff und zur Aufnahme von Kohlendioxid geeignet sein.

Bevorzugt kann vorgesehen sein, dass das medizinische Implantat zur temporären Implantation in Knochenkavitäten geeignet ist.

Bevorzugt kann auch vorgesehen sein, dass der Hohlkörper aus einem für Sauerstoff und Kohlendioxid permeablen Kunststoff gefertigt ist.

Bevorzugt kann ferner vorgesehen sein, dass der Innenraum zur Umgebung des medizinischen Implantats abgeschlossen ist.

Der zumindest eine Kunststoff bildet bevorzugt zumindest bereichsweise eine durchgehende Wandung des Hohlkörpers. Das heißt, dass es Bereiche der Wandung des Hohlkörpers gibt, in denen außer dem zumindest einen Kunststoff aus keinem anderen zusätzlichen Material besteht. Gut durchlässige Netze und Drähte, insbesondere aus Metall, stören dabei nicht. Hierdurch soll sichergestellt werden, dass die Durchlässigkeit des Kunststoffs für Sauerstoff und Kohlendioxid dazu genutzt werden kann, dass die Wandung des Hohlkörpers zumindest in diesen Bereichen ebenfalls für Sauerstoff und Kohlendioxid durchlässig ist.

Bevorzugt kann also vorgesehen sein, dass eine den Innenraum des Hohlkörpers begrenzende Wandung für Sauerstoff und Kohlendioxid durchlässig ist.

Gemäß einer bevorzugten Ausführung der vorliegenden Erfindung kann vorgesehen sein, dass der Hohlkörper röhrenförmig ausgebildet ist, wobei die Fluidzuleitung und die Fluidableitung bevorzugt an einer Stirnseite des Hohlkörpers mit dem Hohlkörper verbunden sind oder an zwei einander gegenüberliegenden Stirnseiten des Hohlkörpers mit dem Innenraum des Hohlkörpers verbunden sind. Röhrenförmige Hohlkörper sind als Abstandshalter insbesondere bei Knochendefekten der langen Röhrenknochen besonders gut geeignet.

Der Hohlkörper kann auch jede andere beliebige Gestalt aufweisen, je nach der Größe und Gestalt des temporär auszufüllenden Knochendefekts.

Das Fluid kann gasförmig oder auch flüssig sein. Es sind auch Gemische aus Flüssigkeiten und Gasen möglich. Als Fluide werden Luft, Sauerstoff, mit Sauerstoff gesättigte Kochsalzlösung, mit Sauerstoff gesättigte Ringer-Lösung, mit Sauerstoff gesättigte Ringer-Lactat-Lösung, mit Sauerstoff gesättigte Phosphatpufferlösung und mit Sauerstoff gesättigtes Perfluordecalin sowie deren Gemische bevorzugt. Wesentlich ist, dass das Fluid Sauerstoff enthält und dass das Fluid Kohlendioxid aufnehmen kann. Es sind daher alle Fluide geeignet, die Sauerstoff und Kohlendioxid transportieren können und diese Stoffe über die permeable Wand des Hohlkörpers mit der Umgebung austauschen können.

Es ist erfindungsgemäß vorgesehen, dass der Hohlkörper oder der zumindest eine Kunststoff des Hohlkörpers einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweist, bevorzugt einen Permeabilitätskoeffizienten für Sauerstoff von größer oder gleich 1 cm³/(m²*d*bar) und einen Permeabilitätskoeffizienten für Kohlendioxid von größer oder gleich 1 cm³/(m²*d*bar) aufweist.

Hierdurch kann eine gute Versorgung der Umgebung des medizinischen Implantats mit Sauerstoff und ein guter Abtransport von Kohlendioxid von der Umgebung des medizinischen Implantats erfolgen und dabei von der Innenseite der Behandlungssituation aus erfolgen.

Der Permeabilitätskoeffizient wird nach DIN 53380-4 (11/2006) bestimmt. Diese betrifft insbesondere die Prüfung von Kunststoffen, hier die Bestimmung der Gasdurchlässigkeit, wobei im Teil 4 der DIN 53380 ein Kohlenstoffdioxid-spezifisches Infrarotabsorptions-Verfahren zur Messung an Kunststoff-Folien und Kunststoff-Formteilen standardisiert wird, das auch auf Sauerstoff übertragbar ist. Derartige Messungen werden beispielsweise von der Firma Mecadi GmbH (Bexbach, Deutschland) durchgeführt und angeboten.

Es kann also bevorzugt vorgesehen sein, dass der Hohlkörper oder der zumindest eine Kunststoff des Hohlkörpers einen Permeabilitätskoeffizienten für Sauerstoff von größer oder gleich 0,5 cm³/(m²*d*bar) und einen Permeabilitätskoeffizienten für Kohlendioxid von gleich/größer 0,5 cm³/(m²*d*bar) gemäß DIN 53380-4 (11/2006) aufweist, bevorzugt einen Permeabilitätskoeffizienten für Sauerstoff von größer oder gleich 1 cm³/(m²*d*bar) und einen Permeabilitätskoeffizienten für Kohlendioxid von größer oder gleich 1 cm³/(m²*d*bar) gemäß DIN 53380-4 (11/2006) aufweist.

Permeabilität bezeichnet allgemein bei Festkörpern die Eigenschaft, Gase und/oder Flüssigkeiten passieren zu lassen. Vorliegend bezieht sich dies auf die Permeabilität der Wandungen des Hohlkörpers und dies bezogen auf molekularen Sauerstoff und molekulares Kohlendioxid in gasförmiger Form. Der Permeabilitätskoeffizient ist eine materialspezifische Konstante und ist ein Maß der Durchlässigkeit für Flüssigkeiten und Gase.

Die Einheit d steht für einen Tag (day).

Es kann ferner vorgesehen sein, dass die Fluidzuleitung und die Fluidableitung derart in den Hohlkörper münden, dass bei einem Durchströmen des Fluids aus der Fluidzuleitung durch den Hohlkörper in die Fluidableitung das Fluid über eine gesamte Innenfläche des Hohlkörpers strömt oder zumindest über 50% der gesamten Innenfläche des Hohlkörpers strömt.

Hierdurch wird beim Durchströmen des Hohlkörpers ein ausreichend langer Kontakt des durchströmenden Fluids mit der Innenwand des Hohlkörpers beziehungsweise des zumindest einen Kunststoffs erreicht, wodurch ein Gasaustausch von Sauerstoff und Kohlendioxid durch die Wand des Hohlkörpers oder des zumindest einen Kunststoffs begünstigt wird.

Wenn der Hohlkörper mit einem Hauptstrang und mit mehreren sich vom Hauptstrang weg erstreckenden Ästen aufgebaut ist, so gelten auch die Äste als Teil der überströmten Oberfläche, auch wenn sich der Hauptteil der Strömung des Fluids entlang des Hauptstrangs an den Ästen vorbei bewegt. Maßgeblich ist hier, dass die inneren Oberflächen der Äste ausgehend vom Hauptstrom des Fluids aus zugänglich sind.

Des Weiteren kann vorgesehen sein, dass eine Einströmöffnung der Fluidzuleitung, mit der die Fluidzuleitung in den Innenraum mündet, räumlich getrennt von einer Ausströmöffnung der Fluidableitung angeordnet ist, wobei die Ausströmöffnung die Einmündung des Innenraums in die Fluidableitung bildet.

Dadurch wird ein Gasaustausch über die gesamte Wandung des Hohlkörpers oder über große Bereiche der Wandung des Hohlkörpers gewährleistet. Die Einströmöffnung ist die Öffnung der Fluidzuleitung, durch die das Fluid aus der Fluidzuleitung in den Innenraum des Hohlkörpers einströmt. Dementsprechend ist die Ausströmöffnung die Öffnung der Fluidableitung, durch die das Fluid in den Fluidableitung aus dem Innenraum des Hohlkörpers ausströmt.

Dabei kann vorgesehen sein, dass die Einströmöffnung der Fluidzuleitung an einem ersten Ende des Hohlkörpers angeordnet ist und die Ausströmöffnung an einem dem ersten Ende gegenüberliegenden zweiten Ende des Hohlkörpers angeordnet ist.

Dadurch wird die Verweilzeit des durchströmenden Fluids an der Innenwand des Hohlkörpers beziehungsweise an der Innenwand des zumindest einen Kunststoffs vergrößert und der Gasaustausch durch den Hohlkörper oder den zumindest einen Kunststoff verbessert.

Ferner kann vorgesehen sein, dass die Fluidzuleitung und die Fluidableitung beide gemeinsam auf einer Seite des Hohlkörpers mit dem Hohlkörper verbunden sind.

Dadurch wird das Einsetzen des Implantats in die Kavität im Knochen des Patienten vereinfacht. Zudem gibt es im Idealfall nur dicht beieinander liegende Zugänge zum Hohlkörper und zur Behandlungssituation, wodurch die Gefahr der mikrobiellen Verkeimung vermindert wird.

Gemäß einer weiteren Ausführung der vorliegenden Erfindung kann vorgesehen sein, dass in der Fluidzuleitung und/oder der Fluidableitung ein keimundurchlässiger aber für Gase durchlässiger Sterilfilter angeordnet ist.

Hierdurch wird die Gefahr einer Infektion des behandelten Patienten und des behandelnden Personals reduziert.

Gemäß einer bevorzugten Ausführung der vorliegenden Erfindung kann vorgesehen sein, dass in der Fluidzuleitung und/oder der Fluidableitung ein Ventil angeordnet ist, insbesondere ein Einwegventil angeordnet ist, wobei das Einwegventil vorzugsweise ein Rückschlagventil ist.

Durch die Verwendung eines Einwegventils oder eines Rückschlagventils ist ein Rückstrom des Fluids sicher ausgeschlossen. Zudem kann mit einem geeigneten einstellbaren Ventil der Druck im Inneren des Hohlkörpers eingestellt werden und so eine bestimmte Form oder eine bestimmte Steifigkeit des medizinischen Implantats erreicht werden.

Gemäß einer bevorzugten Weiterbildung kann vorgesehen sein, dass das Ventil in der Fluidableitung angeordnet ist und als Überdruckventil ausgebildet ist, wobei vorzugsweise der Öffnungsdruck des Überdruckventils einstellbar ist.

Dadurch kann der expandierte Zustand des Hohlkörpers sicher erreicht und auch gehalten werden.

Durch ein Einwegventil in der Fluidableitung kann erreicht werden, dass mit dem Fluid ein Druck im Inneren des Hohlkörpers aufgebaut werden kann und dass das verwendete Fluid nicht in den Hohlkörper zurück gelangen kann. Es kann demzufolge besonders bevorzugt vorgesehen sein, dass in der Fluidableitung ein Einwegventil angeordnet ist.

Es kann auch vorgesehen sein, dass die Fluidzuleitung und die Fluidableitung aus einem für Sauerstoff und Kohlendioxid nicht durchlässigen Material bestehen, vorzugsweise aus einem für Sauerstoff und Kohlendioxid nicht durchlässigen Kunststoff bestehen.

Hierdurch wird verhindert, dass der Sauerstoff zu früh aus dem Fluid austritt. Zudem können die Fluidzuleitung und die Fluidableitung so aus einem kostengünstigeren Kunststoff gefertigt werden.

Ferner kann vorgesehen sein, dass der Hohlkörper oder der zumindest eine Kunststoff des Hohlkörpers mindestens einen antiseptischen Wirkstoff enthält oder mit mindestens einem antiseptischen Wirkstoff beschichtet ist.

Hierdurch können die Oberfläche des medizinischen Implantats und die Umgebung des medizinischen Implantats im Körper des Patienten mit dem mindestens einen antiseptischen Wirkstoff desinfiziert werden. Hierdurch werden Komplikationen bei der Behandlung vermieden.

Des Weiteren kann vorgesehen sein, dass der Hohlkörper ein Hohlzylinder ist oder der Hohlkörper einen Hauptstrang in Form eines Hohlzylinders aufweist, wobei die Fluidzuleitung und die Fluidableitung im Bereich gegenüberliegender Grundflächen des Hohlzylinders in den Hohlzylinder münden, wobei vorzugsweise der Hohlzylinder einen Teil der Fluidzuleitung oder der Fluidableitung koaxial umgibt.

Durch die zylindrische Form kann das Implantat in Knochendefekte von Röhrenknochen eingebracht werden. Zudem kann so sichergestellt werden, dass das Fluid über eine möglichst große Oberfläche der Wandung des Hohlkörpers fließt, so dass der Gasaustausch unterstütz wird.

Bevorzugt kann ferner vorgesehen sein, dass der Hohlkörper als perforierter Metallkörper oder Kunststoffkörper ausgeführt ist, dessen Außenseite mit einer für Sauerstoff und für Kohlendioxid permeablen Kunststoffschicht überdeckt ist.

Dadurch ist es möglich, ein mechanisch belastbares medizinisches Implantat herzustellen und gleichzeitig den Gasaustausch durch die Wandung des Hohlkörpers zu gewährleisten.

Zur Verbesserung der Formbarkeit des Hohlkörpers kann vorgesehen sein, dass der Hohlkörper einen für Sauerstoff und Kohlendioxid permeablen und plastisch verformbaren Kunststoffmantel aufweist, wobei vorzugsweise der Kunststoffmantel spiralförmige und/oder netzartig angeordnete Metalldrähte enthält.

Dadurch kann das medizinische Implantat je nach anatomischen Gegebenheiten geformt werden und behält gegebenenfalls auf Grund der Steifheit der Metalldrähte die gewählte Form bei.

Um zusätzliche Behandlungsmöglichkeiten zu eröffnen kann vorgesehen sein, dass am Hohlkörper eine Leitung mit mehreren Öffnungen zur Abgabe eines Wirkstoffs angeordnet ist, wobei die Leitung mit einer Wirkstoffzuleitung verbunden ist, wobei bevorzugt die Summe der freien Querschnittsflächen der Öffnungen kleiner ist als der freie Querschnitt der Leitung und der Wirkstoffzuleitung.

Mit dieser Ausgestaltungsform ist es möglich, lokal pharmazeutische Wirkstofflösungen zu applizieren. Als pharmazeutische Wirkstoffe kommen hierbei Antibiotika, Antiseptika, Antiphlogistika, Bisphosphonate, Wachstumsfaktoren, Bakteriophagen, Lysostaphin und Muramidasen in Betracht. Daneben ist es auch möglich, Zellsuspensionen durch die Wirkstoffzuleitung in die Umgebung des Implantats einzubringen und eine Besiedlung der das medizinische Implantat umgebenen Strukturen zu ermöglichen.

Durch die Leitung mit den Öffnungen ist es auch möglich, antiseptische oder antibiotische Lösungen durch die Öffnungen zur Umgebung des medizinischen Implantats zu pressen. Durch die Leitung ist auch ein antimikrobieller Schutz der Oberfläche des medizinischen Implantats möglich. Es ist auch möglich, durch die Leitung flüssige oder gelartige antibiotische oder antiseptische pharmazeutische Zubereitungen einzubringen, die durch Diffusion in die Umgebung migrieren können und so zur Behandlung des Patienten im Bereich des medizinischen Implantats verwendet werden können.

Es kann auch vorgesehen sein, dass der zumindest eine Kunststoff ein elastischer und/oder plastischer, nicht biodegradierbaren Kunststoff ist, wobei bevorzugt der zumindest eine Kunststoff ausgewählt ist aus Polyurethan, Ethylen-Propylen-Dien-Kautschuk EPDM und Silikon, oder der zumindest eine Kunststoff ein elastischer und/oder plastischer, biodegradierbaren Kunststoff ist, wobei bevorzugt der zumindest eine Kunststoff ausgewählt ist aus Gelatine, vernetztem Kollagen und vernetztem Albumin.

Durch diese beiden Möglichkeiten kann ein gut wieder entfernbares medizinisches Implantat bereitgestellt werden (nicht biodegradierbarer Kunststoff) oder das medizinische Implantat kann teilweise oder vollständig im Körper abgebaut werden (biodegradierbarer Kunststoff) und muss nicht oder nicht vollständig wieder entfernt werden.

Des Weiteren kann vorgesehen sein, dass der Hohlkörper durch Änderung des Drucks in seinem Innenraum gegenüber der umgebenden Atmosphäre expandierbar und/oder zusammenziehbar ist.

Bei der Verwendung von eines nicht formstabilen, elastischen Implantatmaterials kann die gewählte Gestalt des Hohlkörpers durch einen geeigneten Innendruck des Hohlkörpers gewährleistet werden. Zudem können das Einsetzen und das Entfernen des medizinischen Implantats so vereinfacht werden.

Gemäß einer bevorzugten Variante der vorliegenden Erfindung kann auch vorgesehen sein, dass der Hohlkörper einen Hauptstrang und mehrere seitlich davon abgehende Äste aufweist, wobei der Innenraum des Hohlkörpers sich im Hauptstrang und in den Ästen erstreckt und die Fluidzuleitung und die Fluidableitung mit dem Hauptstrang verbunden sind, wobei vorzugsweise zumindest die Äste oder Wandungen der Äste aus dem Kunststoff bestehen oder mit dem Kunststoff aufgebaut sind.

Bevorzugt weist der Innenraum in den Ästen einen geringeren Querschnitt auf als in dem Hauptstrang.

Hierdurch kann die Oberfläche des Hohlkörpers vergrößert werden und so eine größere Menge von Zellen versorgt werden, insbesondere extrakorporal versorgt werden.

Ferner kann vorgesehen sein, dass der Hohlkörper aus zumindest einem resorbierbaren und/oder biodegradierbaren Material besteht.

Hierdurch kann der Hohlkörper im Körper verbleiben und dort abgebaut werden, ohne dass er wieder aus dem Körper entfernt werden muss. Die Fluidzuleitung und die Fluidableitung können hierzu einfach abgetrennt werden.

Diese Varianten ermöglichen die extrakorporale Vermehrung von Zellen in Hohlräumen von Zellkulturgefäßen. Als wachstumsfördernde Substanzen kommt beispielsweise Tricalciumphosphat oder ähnlich wirkende Substanzen in Frage. Die so vermehrten Zellen können dann zusammen mit dem medizinischen Implantat oder auch ohne das medizinische Implantat implantiert werden. Wenn der Hohlkörper aus einer vernetzten Gelatine oder Biopolymeren aufgebaut wird, kann das medizinische Implantat bis auf die Anschlüsse abbaubar sein. Das Gleiche ist auch möglich, wenn der Hohlkörper aus abbaubaren Hohlfasern aufgebaut ist.

Es kann vorgesehen sein, dass der Hohlkörper oder das gesamte medizinische Implantat als dreidimensionale Struktur faltbar ist. Hierdurch kann das medizinische Implantat an die Form der zu behandelnden Kavität anpassbar gestaltet werden.

Das medizinische Implantat kann als belüftbares Scaffold zur Zellkultivierung verwendet werden. Ein "Scaffold" bezeichnet im Tissue Engineering (TE) (engl. für Gewebekonstruktion bzw. Gewebezüchtung) einen Überbegriff für die künstliche Herstellung von biologischem Gewebe durch eine gerichtete Kultivierung von Zellen, um damit kranke Gewebe bei einem Patienten zu ersetzen oder zu regenerieren. Hierfür ist keine medizinische Behandlung eines Patienten erforderlich, da diese erst später erfolgt mit Hilfe der künstlich hergestellten Zellen. Das medizinische Implantat kann dann als Trägerstruktur für Zellkulturen verwendet werden. Die der vorliegenden Erfindung zugrundeliegenden Aufgaben werden auch gelöst durch ein Knochendefektbehandlungssystem aufweisend ein solches erfindungsgemäßes medizinisches Implantat und das Fluid, wobei das Fluid Sauerstoff enthält und zur Aufnahme von Kohlendioxid geeignet ist.

Hierdurch wird ein vollständiges System (Knochendefektbehandlungssystem) bereitgestellt, bei dem das Fluid bereits auf das medizinische Implantat beziehungsweise beide auf die Behandlungssituation abgestimmt sein kann.

Dabei kann vorgesehen sein, dass das Fluid ausgewählt ist aus Luft, Sauerstoff, mit Sauerstoff gesättigter Kochsalzlösung, mit Sauerstoff gesättigter Ringer-Lösung, mit Sauerstoff gesättigter Ringer-Lactat-Lösung, mit Sauerstoff gesättigter Phosphatpufferlösung und mit Sauerstoff gesättigtem Perfluordecalin oder einem Gemisch aus zumindest zwei der genannten Gase oder Flüssigkeiten.

Diese Fluide sind gut zur Abgabe von Sauerstoff und zur Aufnahme von Kohlendioxid geeignet. Zudem sind viele dieser Fluide gesundheitlich unbedenklich.

Es ist vorgesehen, dass das Knochendefektbehandlungssystem ein Knochenersatzmaterial aufweist, wobei das Knochenersatzmaterial auf der äußeren Oberfläche des Hohlkörpers aufgetragen ist oder auf die äußere Oberfläche des Hohlkörpers aufbringbar ist.

Hiermit wird ein weiter vervollständigtes Knochendefektbehandlungssystem bereitgestellt, das alle Bestandteile enthält, die zur Behandlung eines Knochendefekts notwendig und hilfreich sind.

Dabei kann wiederum vorgesehen sein, dass das Knochenersatzmaterial ausgewählt ist aus einem nicht biodegradierbaren, einem teildegradierbaren, einem vollständig biodegradierbaren Knochenersatzmaterial und deren Mischungen.

Diese Mischungen sind besonders gut mit dem erfindungsgemäßen medizinischen Implantat einsetzbar.

Des Weiteren kann vorgesehen sein, dass das Knochenersatzmaterial ausgewählt ist aus autologem Knochengewebe, allogenem Knochengewebe, Hydroxylapatit, Carbonatapatit, β-Tricalciumphosphat, α-Tricalciumphosphat, Calcium-Dihydrat, Brushit, Monetit und deren Mischungen, oder das Knochenersatzmaterial lebende Zellen enthält und/oder mit lebenden Zellen auf seiner Oberfläche besiedelt ist.

Diese Knochenersatzmaterialien lassen sich besonders gut mit dem erfindungsgemäßen medizinischen Implantat verwenden. Eine Versorgung dieser Knochenersatzmaterialien mit Sauerstoff und eine Entsäuerung durch Kohlendioxidaufnahme wirken sich bei diesen Knochenersatzmaterialien besonders gut für die Behandlungssituation aus.

Es ist besonders vorteilhaft, wenn das medizinische Implantat mit autologem Knochengewebe und besonders bevorzugt mit autologer Spongiosa kombiniert wird. Durch den Sauerstoffeintrag über den Hohlkörper werden die Zellen des autologen Knochengewebes mit Sauerstoff versorgt und gleichzeitig wird das beim Stoffwechsel der Zellen freigesetzte Kohlendioxid abgeführt. Sauerstoffmangel kann zur Behinderung der Atmung der Zellen führen, so dass diese zuerst beginnen zu gären und dann abzusterben. Gärungsprozesse können eine lokale Übersäuerung bewirken. Weiterhin wird eine lokale Übersäuerung durch den Abtransport des gebildeten Kohlendioxids verhindert.

Die der vorliegenden Erfindung zugrundeliegenden Aufgaben bezüglich eines nicht medizinischen Verfahrens werden gelöst durch ein Verfahren zum Begasen einer Oberfläche eines medizinischen Implantats, insbesondere eines erfindungsgemäßen medizinischen Implantats, bevorzugt mit einem erfindungsgemäßen Knochendefektbehandlungssystem, gekennzeichnet durch die folgenden Schritte:
A) Einleiten eines Fluids enthaltend Sauerstoff in einen Innenraum eines Hohlkörpers des medizinischen Implantats;
B) Abgeben von Sauerstoff aus dem Fluid durch einen den Innenraum des Hohlkörpers begrenzenden Kunststoff hindurch an die Umgebung des Hohlkörpers;
C) Aufnehmen von Kohlendioxid aus der Umgebung des Hohlkörpers durch den, den Innenraum begrenzenden Kunststoff hindurch in das Fluid;
D) Hindurchleiten des Fluids durch den Innenraum des Hohlkörpers und Ableiten des Fluids aus dem Innenraum des Hohlkörpers, wobei das Verfahren nicht zur medizinischen Behandlung eines menschlichen oder tierischen Körpers durchgeführt wird.

Die Schritte B) und C) laufen vorzugsweise gleichzeitig ab. Zudem erfolgt der Gasaustausch in den Schritten B) und C) bevorzugt auch schon während Schritt A) ab und die ganze Zeit während Schritt D).

Als Fluid können Luft oder Sauerstoff verwendet werden. Im Rahmen der Erfindung ist es auch, wenn anstelle von Luft oder Sauerstoff als das Fluid mit Sauerstoff gesättigte Spülflüssigkeiten, wie zum Beispiel physiologische Kochsalzlösung, Ringer-Lösung oder Ringer-Lactat-Lösung, in das medizinische Implantat eingebracht werden, insbesondere durch die Fluidzuleitung. Es ist weiterhin auch möglich, als Sauerstoffträger perfluoriertes Decalin oder andere perfluorierte Flüssigkeiten als das Fluid zu verwenden, in denen Sauerstoff löslich ist. Durch die Fluidableitung können diese Fluide wieder aus dem Hohlkörper ausgeleitet werden.

Bei erfindungsgemäßen Verfahren ist vorgesehen, dass das Verfahren nicht zur medizinischen Behandlung eines menschlichen oder tierischen Körpers durchgeführt wird.

Es kann bevorzugt vorgesehen sein, dass das Verfahren außerhalb eines menschlichen oder eines tierischen Körpers durchgeführt wird.

Es kann auch vorgesehen sein, dass das erfindungsgemäße Verfahren nicht von einem Arzt oder einem Mediziner durchgeführt wird.

Erfindungsgemäße medizinische Implantate erlauben neben der Behandlung von Knochendefekten auch eine extrakorporale Vermehrung von Zellen. Hierzu können Zellen und Nährstoffe auf die äußere Oberfläche des Kunststoffs oder des Hohlkörpers aufgebracht werden. Diese werden dann über das Fluid mit Sauerstoff versorgt, während Kohlendioxid aus den wachsenden Zellkulturen entfernt wird.

Ferner kann vorgesehen sein, dass vor Schritt A) der Hohlkörper in einen Hohlraum eingeführt wird und ein Knochenersatzmaterial auf die Oberfläche des medizinischen Implantats aufgebracht wird und/oder in den Hohlraum zwischen das medizinische Implantat und den Hohlraum begrenzende Innenwände eingebracht wird.

Der Hohlraum ist bevorzugt keine Kavität eines menschlichen oder tierischen Körpers.

Durch das Einführen in den Hohlraum werden die Vorteile des Verfahrens voll zu Geltung gebracht. Der Gasaustausch zwischen dem Hohlraum und dem Innenraum des Hohlkörpers bewirkt die erfindungsgemäßen Vorteile.

Schließlich kann auch vorgesehen sein, dass das Verfahren zur extrakorporalen Vermehrung von Zellen, insbesondere von Knochenzellen, angewendet wird, wobei vor Schritt A) die Zellen auf der äußeren Oberfläche des Hohlkörpers aufgebracht werden, bevorzugt zusammen mit einer Nährstofflösung und/oder wachstumsfördernden Substanzen auf der äußeren Oberfläche des Hohlkörpers aufgebracht werden.

Hierdurch können bereits versorgte Zellen mit dem medizinischen Implantat zusammen in den Knochendefekt eingebracht werden, um die Heilung zu beschleunigen und den Heilungserfolg zu verbessern.

Das medizinische Implantat kann so zum Belüften und Vermehren einer Zellkultur für Knochenzellen an der Oberfläche des Hohlkörpers extrakorporal verwendet werden. Mit der gewachsenen Zellkultur versehen kann das medizinische Implantat anschließend implantiert werden und dann noch weiter im Inneren des Körpers belüftet werden, um ein weiteres Vermehren und Anwachse der Zellen im Knochendefekt zu fördern.

Das medizinische Implantat muss nicht entfernt werden, wenn der Hohlkörper biodegradierbar beziehungsweise vom Körper abbaubar ist. Die Anschlüsse beziehungsweise die Fluidzuleitung und die Fluidableitung können dann einfach zu einem gewünschten Zeitpunkt abgeschnitten werden und der Hohlkörper und die wachsenden Zellen verbleiben im Körper und werden resorbiert.

Der Erfindung liegt die überraschende Erkenntnis zugrunde, dass es durch das medizinische Implantat gelingt, den Aufbau der umgebenden Knochenstruktur zu unterstützen, indem eine Versorgung der Umgebung des medizinischen Implantats mit Sauerstoff erfolgt und eine Übersäuerung der Umgebung des medizinischen Implantats durch Kohlendioxid beziehungsweise Kohlensäure vermieden wird. Aus diesen Gründen kann eine schnellere und wirkungsvollere Ossifikation gelingen, so dass der Heilungserfolg schneller eintreten kann. Der besondere Vorteil ist dabei insbesondere auch darin zu sehen, dass die vom Blutkreislauf abgewandte Innenseite des Knochens, die normalerweise vom Blutkreislauf nur schlecht mit Sauerstoff versorgt werden kann, mit dem erfindungsgemäßen medizinischen Implantat besser versorgt werden kann, da dieses direkt an Innenseite der Behandlungssituation angrenzend positionierbar ist. Durch die Verwendung des für Sauerstoff und Kohlendioxid durchlässigen Kunststoffs kann zuverlässig und gleichmäßig Sauerstoff aus einem den Hohlkörper durchströmenden Fluid an die Umgebung des Hohlkörpers abgegeben werden und gleichzeitig Kohlendioxid aus der Umgebung des Hohlkörpers aufgenommen werden, um ein für die Heilung des Knochendefekts geeignetes Klima zu schaffen.

Weiterhin ist vorteilhaft, dass nach Beendigung der Implantationsphase der Hohlkörper durch Anlegen von Unterdruck sein Volumen verringern kann und dadurch die Entfernung des Hohlkörpers durch eine kleine Öffnung erfolgen kann. Der Abtransport des Kohlendioxids verhindert eine Versäuerung der Zellen.

Nach erfolgtem Einwachsen von Osteoblasten in das Knochenersatzmaterial oder Anwachsen von Osteoblasten des Knochenersatzmaterials, wird das temporäre Implantat entfernt. Voraussetzung ist dafür, dass eine ausreichende Neovaskularisierung erfolgt ist, so dass der Transport von Sauerstoff und Nährstoffen sowie der Abtransport von Kohlendioxid gewährleistet sind. Das bedeutet, das temporäre Implantat hatte nur die Versorgung mit Sauerstoff und den Abtransport von Kohlendioxid temporär übernommen, bis die patienteneigenen Blutgefäße nachgebildet waren. Der nach der Entfernung des temporären Implantats ist verbliebene Hohlraum entweder im Durchmesser so klein, dass ein spontanes Einwachsen des Knochengewebes möglich ist oder es wird der Hohlraum mit Knochenersatzmaterial, bevorzugt granulärem Knochenersatzmaterial, aufgefüllt.

Ein beispielhaftes erfindungsgemäßes medizinisches Implantat kann beispielsweise aufweisen:
a) zumindest einen für Sauerstoff und Kohlendioxid permeablen Hohlkörper, der für Flüssigkeiten undurchlässig ist,
b) mindestens eine Fluidzuleitung für Fluide, die mit dem zumindest einen permeablen Hohlkörper fluiddurchlässig verbunden ist,
c) mindestens eine Fluidableitung für Fluide, die mit dem zumindest einen permeablen Hohlkörper fluiddurchlässig verbunden ist,
d) mindestens ein Fluid, das Sauerstoff enthält und Kohlendioxid aufnehmen kann, wobei das Fluid durch die Fluidzuleitung in den Innenraum des zumindest einen Hohlkörpers geleitet wird und von dort durch die Fluidableitung aus dem Innenraum des zumindest einen Hohlkörpers heraus transportiert wird,
e) wobei durch den Innenraum des zumindest einen Hohlkörpers das mindestens eine Fluid so geleitet wird, dass Sauerstoff aus dem mindestens einen Fluid durch die Wand des zumindest einen Hohlkörpers in die Umgebung des zumindest einen Hohlkörpers und Kohlendioxid von der Umgebung in das mindestens eine Fluid im Innenraum des zumindest einen Hohlkörpers gelangen beziehungsweise permeieren können.

Ein beispielhaftes erfindungsgemäßes Knochenersatzmaterialsystem kann zusammengesetzt sein aus einem solchen medizinischen Implantat und einem nicht biodegradierbaren und/oder einem teildegradierbaren und/oder vollständig biodegradierbaren Knochenersatzmaterial, und deren Mischungen.

Im Folgenden werden drei weitere Ausführungsbeispiele der Erfindung anhand von dreizehn schematisch dargestellten Figuren erläutert, ohne jedoch dabei die Erfindung zu beschränken. Dabei zeigt:
Figur 1: eine schematische perspektivische Ansicht auf ein erstes beispielhaftes erfindungsgemäßes medizinisches Implantat;
Figur 2: eine schematische Querschnittansicht des ersten beispielhaften medizinischen Implantats nach Figur 1;
Figur 3: eine Ausschnittvergrößerung der Querschnittansicht nach Figur 2, die die rückseitigen Anschlüsse zeigt;
Figur 4: eine schematische Querschnittansicht des ersten medizinischen Implantats nach den Figuren 1 bis 3 in der die Strömungsverhältnisse und die Abgabe von Sauerstoff durch spitze Pfeile angedeutet ist;
Figur 5: eine schematische perspektivische Ansicht auf ein zweites beispielhaftes erfindungsgemäßes medizinisches Implantat;
Figur 6: eine schematische Querschnittansicht des zweiten beispielhaften medizinischen Implantats nach Figur 5;
Figur 7: eine schematische Querschnittansicht des zweiten beispielhaften medizinischen Implantats nach den Figuren 5 und 6, wobei die Strömungsverhältnisse und die Abgabe von Sauerstoff durch spitze Pfeile angedeutet ist;
Figur 8: eine Ausschnittvergrößerung der Querschnittansichten nach den Figuren 6 und 7, die die Vorderseite des medizinischen Implantats zeigt, wobei die Strömungsverhältnisse sowie die Abgabe von Sauerstoff und die Aufnahme von Kohlendioxid durch spitze Pfeile angedeutet sind;
Figur 9: eine Ausschnittvergrößerung der Querschnittansichten nach den Figuren 6 und 7, die die rückseitigen Anschlüsse zeigt;
Figur 10: eine schematische perspektivische Ansicht auf ein drittes beispielhaftes erfindungsgemäßes medizinisches Implantat;
Figur 11: eine schematische perspektivische Ansicht das dritte beispielhafte medizinische Implantat in einem gefalteten Zustand;
Figur 12: eine schematische Querschnittansicht des dritten beispielhaften medizinischen Implantats nach Figur 10, wobei die Strömungsverhältnisse sowie die Abgabe von Sauerstoff und die Aufnahme von Kohlendioxid durch spitze Pfeile angedeutet sind; und
Figur 13: eine Ausschnittvergrößerung der Querschnittansicht nach Figur 12, die die Anschlüsse zeigt.

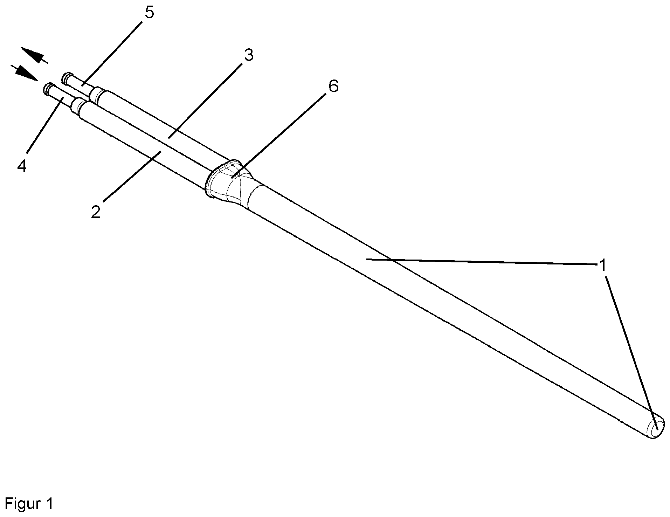

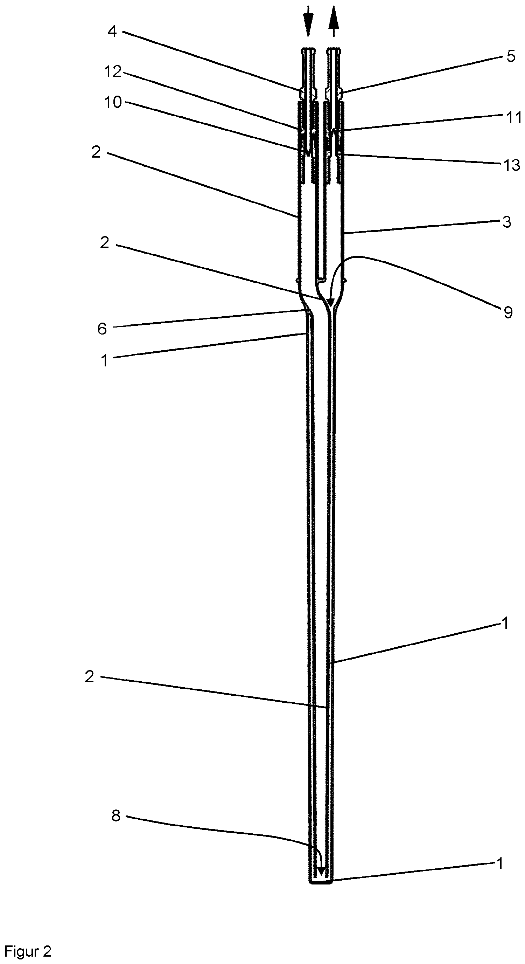

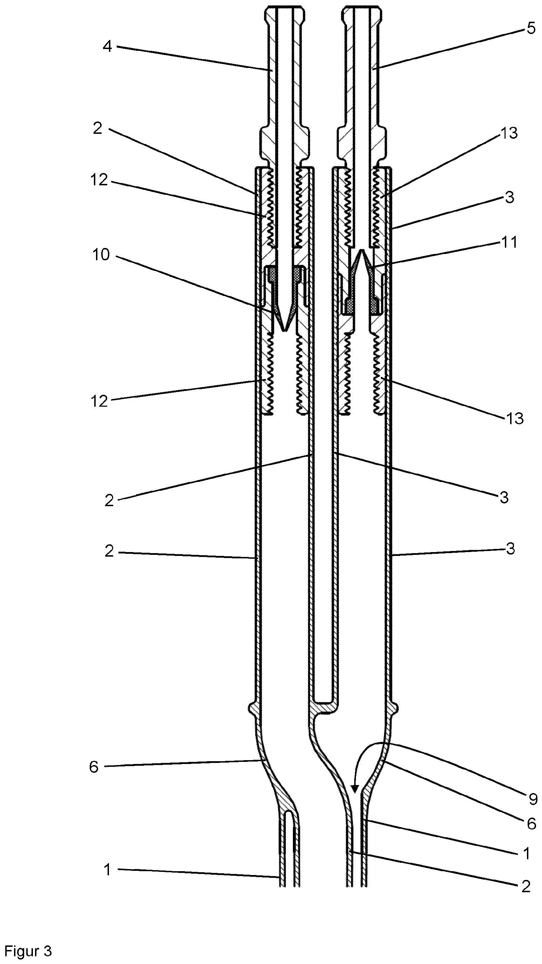

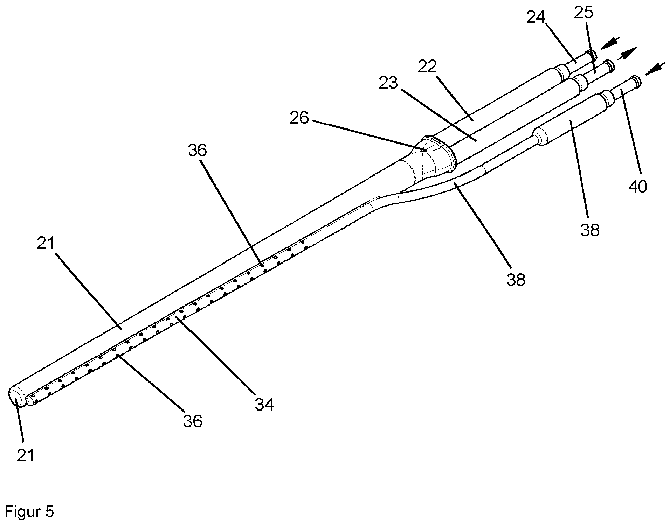

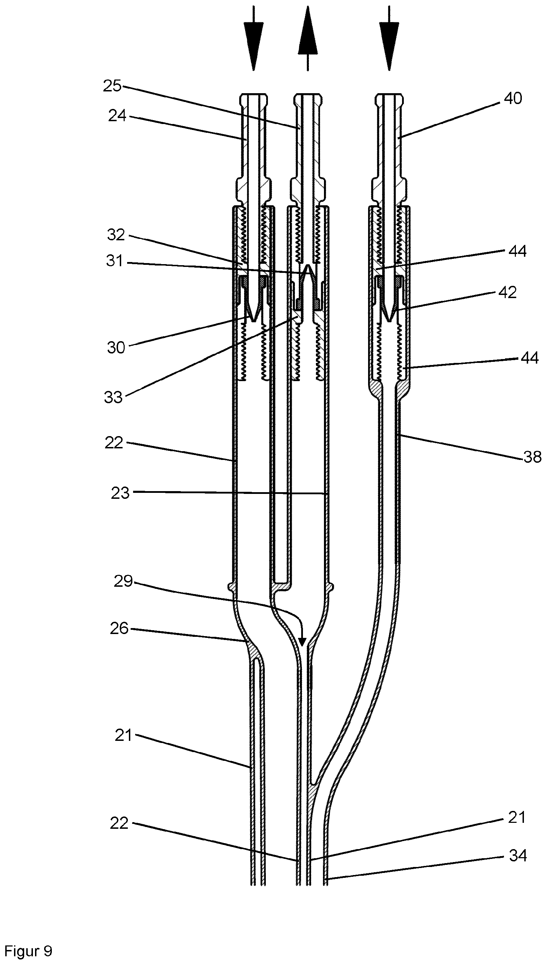

In den Figuren 2 bis 4 und 6 bis 9 ist die Vorderseite des jeweiligen medizinischen Implantats nach unten und die Rückseite nach oben ausgerichtet. In Figur 1 ist die Vorderseite des ersten beispielhaften medizinischen Implantats nach unten rechts ausgerichtet und die Rückseite nach oben links. In Figur 5 ist die Vorderseite des zweiten beispielhaften medizinischen Implantats nach unten links ausgerichtet und die Rückseite nach oben rechts.

In den Figuren 1 bis 4 sind also Abbildungen des ersten erfindungsgemäßen medizinischen Implantats gezeigt und in den Figuren 5 bis 9 Abbildungen des zweiten erfindungsgemäßen medizinischen Implantats.

Das erste erfindungsgemäße medizinische Implantat kann einen Hohlkörper 1 aus einem elastisch oder plastisch verformbaren Kunststoff aufweisen oder einen Hohlkörper 1 aufweisen, dessen Wandungen zumindest bereichsweise aus einem Kunststoff bestehen. Der Hohlkörper 1 kann ein einseitig geschlossenes Zylinderrohr sein. Der Hohlkörper 1 kann beispielsweise aus einem biokompatiblen Kunststoff bestehen. Der Hohlkörper 1 kann für Flüssigkeiten undurchlässig sein. Im Inneren des Hohlkörpers 1 kann ein Innenraum angeordnet sein.

Das für den Hohlkörper 1 zumindest bereichsweise verwendete Material kann für molekularen Sauerstoff und für Kohlendioxid durchlässig sein. Der Hohlkörper 1 beziehungsweise das Material, aus dem der Hohlkörper 1 gefertigt ist, kann hierzu einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweisen. Der Permeabilitätskoeffizient wird dabei nach DIN 53380-4 (11/2006) bestimmt.

Zum Einleiten eines Fluids kann ein Innenraum des Hohlkörpers 1 mit einer Fluidzuleitung 2 aus Kunststoff verbunden sein. Zum Ableiten des Fluids aus dem Hohlkörper 1 heraus kann der Innenraum des Hohlkörpers 1 mit einer Fluidableitung 3 aus Kunststoff verbunden sein. Die Fluidzuleitung 2 und die Fluidableitung 3 können zumindest bereichsweise flexibel und beweglich sein. Die Fluidzuleitung 2 kann an ihrer Rückseite (in Figur 1 links oben, in den Figuren 2 bis 4 oben) einen Anschluss 4 aufweisen, mit dem die Fluidzuleitung 2 an eine Fluidquelle (nicht gezeigt) angeschlossen werden kann. Ebenso kann die Fluidableitung 3 an ihrer Rückseite einen Anschluss 5 aufweisen, mit dem die Fluidableitung 3 an eine Aufnahme oder ein Abfluss für verbrauchtes Fluid angeschlossen werden kann.

Die Fluidzuleitung 2 und die Fluidableitung 3 können in einer Verbindung 6 zusammengeführt sein, in der die Fluidzuleitung 2 koaxial in die Fluidableitung 3 beziehungsweise in den Hohlkörper 1 geführt sein kann. Die Fluidzuleitung 2 kann koaxial in dem Hohlkörper 1 angeordnet sein. Die Fluidzuleitung 2 kann in dem Hohlkörper 1 bis fast an das vordere geschlossene Ende des Hohlkörpers 1 geführt sein und dort durch eine Einströmöffnung 8 in den Hohlkörper 1 münden. Die Fluidzuleitung 2 kann über die Einströmöffnung 8 in den vorderen Teil des Innenraums des Hohlkörpers 1 münden. Die Fluidableitung 3 kann über eine Ausströmöffnung 9 an der gegenüberliegenden Rückseite des Innenraums des Hohlkörpers 1 mit dem Innenraum des Hohlkörpers 1 verbunden sein. Hierdurch wird sichergestellt, dass das Fluid entlang der Oberfläche der Wandung des gesamten Hohlkörpers 1 strömen kann und dadurch ein Gasaustausch von Sauerstoff und Kohlendioxid durch die Wandung auf der gesamten Länge des Hohlkörpers 1 erfolgen kann. Die Ausströmöffnung 9 kann durch die Verbindung 6 begrenzt sein.

In der Fluidzuleitung 2 kann ein Ventil 10 in Form eines Lippenventils angeordnet sein, das eine Strömung des Fluids in Richtung des Hohlkörpers 1 ermöglicht aber eine Strömung des Fluids in der Richtung vom Hohlkörper 1 weg verhindert. Das Ventil 10 wirkt dann als Einwegventil. In der Fluidableitung 3 kann ein Ventil 11 in Form eines Lippenventils angeordnet sein, das eine Strömung des Fluids in Richtung des Hohlkörpers 1 verhindert aber eine Strömung des Fluids in der Richtung vom Hohlkörper 1 weg erlaubt. Das Ventil 11 wirkt dann als Einwegventil. Das Ventil 11 in der Fluidableitung 3 kann dazu ausgelegt sein, dass es ab einem Mindestdruck des Fluids öffnet. Bevorzugt ist der Mindestdruck am Ventil 11 in der Fluidableitung 3 einstellbar. Der Mindestdruck kann dabei so gewählt werden, dass der Druck des Fluids dazu ausreicht, den Hohlkörper 1 in eine gewünschte äußere Form zu bringen.

Die Ventile 10, 11 können über Ventilgehäuse 12, 13 mit der Fluidzuleitung 2 und der Fluidableitung 3 verbunden sein. Hierzu kann die Fluidzuleitung 2 auf das Ventilgehäuse 12 gesteckt sein und gegebenenfalls zusätzlich befestigt sein. Ebenso kann die Fluidableitung 3 auf Ventilgehäuse 13 gesteckt sein und dort gegebenenfalls zusätzlich befestigt sein.

Der Anschluss 4 kann an seiner Rückseite die Form eines Luer-Lock-Adapters haben. Ebenso kann der Anschluss 5 an seiner Rückseite die Form eines Luer-Lock-Adapters haben. Durch die Anschlüsse 4, 5 kann das Fluid eingespeist und abgeleitet werden. Die Anschlüsse 4, 5 können in die Ventilgehäuse 12, 13 eingeschraubt sein.

Das Ventilgehäuse 12 kann zur Fixierung des Ventils 10 zweiteilig aufgebaut sein. Das Ventilgehäuse 12 kann über ein Innengewinde mit einem Außengewinde des Anschlusses 4 verbunden sein. Das Ventilgehäuse 13 kann zur Fixierung des Ventils 11 zweiteilig aufgebaut sein. Das Ventilgehäuse 13 kann über ein Innengewinde mit einem Außengewinde des Anschlusses 5 verbunden sein. Alle Verbindungen können gasdicht und Druckdicht ausgeführt sein.

Die Fluidzuleitung 2 kann auf das Ventilgehäuse 12 aufgesteckt sein. Es kann vorgesehen sein, dass die Fluidzuleitung 2 dort mit einer Krimphülse (nicht gezeigt) druckdicht und gasdicht befestigt wird. Die Fluidableitung 3 kann auf das Ventilgehäuse 13 aufgesteckt sein. Es kann vorgesehen sein, dass die Fluidableitung 3 dort mit einer Krimphülse (nicht gezeigt) druckdicht und gasdicht befestigt wird.

Der Hohlkörper 1 kann in einen Hohlraum eingebracht werden. Der Hohlkörper 1, beziehungsweise das medizinische Implantat, kann so den Hohlraum mechanisch stützen und stabilisieren. Wenn das medizinische Implantat nicht mehr benötigt wird, kann der Hohlkörper 1 durch Anlegen eines Unterdrucks komprimiert werden, beispielsweise indem er evakuiert wird. Der Hohlkörper 1 kann dann wieder bequem aus dem Hohlraum entfernt werden. Insbesondere wenn der Hohlraum eine Kavität in einem Knochen ist, kann dieser Knochendefekt so schonend behandelt werden. Alternativ kann der Hohlkörper 1 auch innerhalb des Körpers abgebaut werden, wenn er aus einem biodegradierbaren Material gefertigt ist.

Im Betrieb kann das Fluid durch den Anschluss 4 in das medizinische Implantat eingespeist werden (so wie das in den Figuren 1, 2 und 4 durch den in den Anschluss 4 hineinweisenden spitzen Pfeil angedeutet ist). Das Fluid kann durch die Fluidzuleitung 2 strömen und bei ausreichendem Druck das Ventil 10 öffnen. Dann kann das Fluid durch die Einströmöffnung 8 in den Hohlkörper 1 und durch den Hohlkörper 1 fließen. Das Fluid kann durch die Fluidableitung 3 bis zum zunächst geschlossenen Ventil 11 strömen. Dabei kann sich im Inneren des Hohlkörpers 1 ein Druck aufbauen. Sobald der Druck am Ventil 11 in der Fluidableitung 3 ausreicht, öffnet sich das Ventil 11 und das Fluid kann durch die Fluidableitung 3 und den Anschluss 5 abfließen (so wie das in den Figuren 1, 2 und 4 durch den vom Anschluss 5 wegweisenden spitzen Pfeil angedeutet ist).

In dem Fluid ist Sauerstoff enthalten. Das Fluid kann durch die Wandung des Hohlkörpers 1 Sauerstoff an die Umgebung des Hohlkörpers 1 abgeben. Gleichzeitig kann das strömende Fluid Kohlendioxid aus der Umgebung des Hohlkörpers 1, das durch die Wandung des Hohlkörpers 1 in den Innenraum diffundiert, aufnehmen, und aus dem medizinischen Implantat durch den Anschluss 5 ableiten. Hierdurch kann die Umgebung des Hohlkörpers 1 mit Sauerstoff versorgt werden und durch die Aufnahme von Kohlendioxid eine Übersäuerung der Umgebung des Hohlkörpers 1 verhindert werden.

In der Fluidzuleitung 2 und/oder in der Fluidableitung 3 können keimundurchlässige aber für das Fluid durchlässige Sterilfilter (nicht gezeigt) angeordnet sein. Insbesondere wenn das Fluid gasförmig ist, kann diese Maßnahme problemlos eingesetzt werden. Wenn das Fluid flüssig ist, muss darauf geachtet werden, dass die Sterilfilter den Fluss des Fluids nicht zu stark hemmen. Das Fluid kann mit dem Sterilfilter von Keimen befreit werden, die ansonsten in den Hohlkörper 1 gelangen könnten und/oder die vom Hohlkörper 1 durch den Anschluss 5 abgeleitet werden könnten. Hierdurch wird die Gefahr einer Infektion des behandelten Patienten und des behandelnden Personals reduziert. Bevorzugt kann der Sterilfilter in der Fluidzuleitung 2 oder der Fluidableitung 3 in Strömungsrichtung hinter dem Ventil 10 oder dem Ventil 11 angeordnet sein oder die Sterilfilter in der Fluidzuleitung 2 und in der Fluidableitung 3 hinter den Ventilen 10, 11 angeordnet sein. Es sind auch andere Verfahren und Möglichkeiten zur Sterilisierung des Fluids möglich. Beispielsweise kann das Fluid mit einer Strahlung sterilisiert werden.

Es kann auch vorgesehen sein, dass der Hohlkörper 1 sowie die angrenzenden Bereiche der Fluidzuleitung 2 und der Fluidableitung 3 mit einer antiseptischen Substanz beschichtet sind oder eine lösbare antiseptische Substanz in dem Material des Hohlkörpers 1 enthalten ist, um eine Infektion zu verhindern.

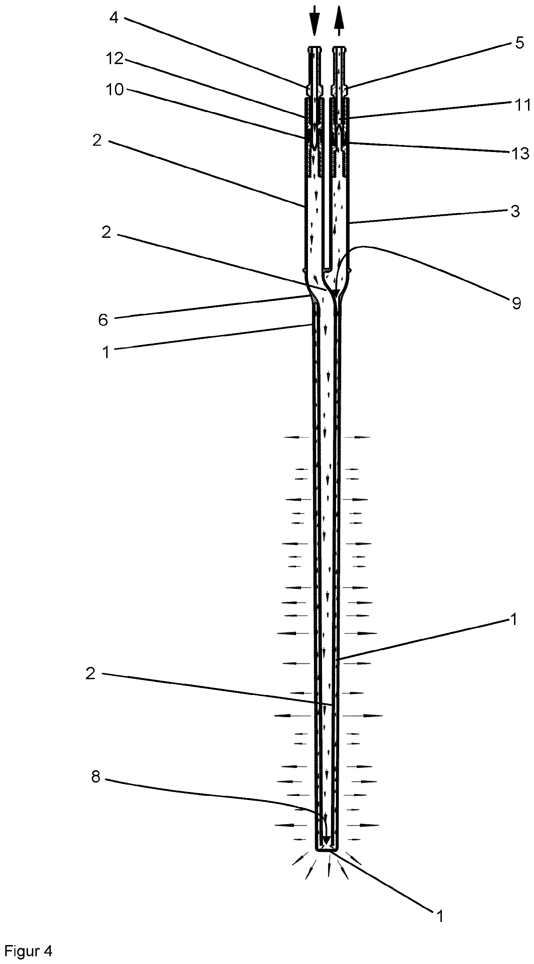

Die spitzen Pfeile in Figur 4 im Inneren des Hohlraums 1, der Fluidzuleitung 2 und der Fluidableitung 3 deuten die Fließrichtung des Fluids während des Betriebs an. Des Weiteren deuten die spitzen Pfeile im Bereich um den Hohlraum 1 die Abgabe von Sauerstoff aus dem Fluid an.

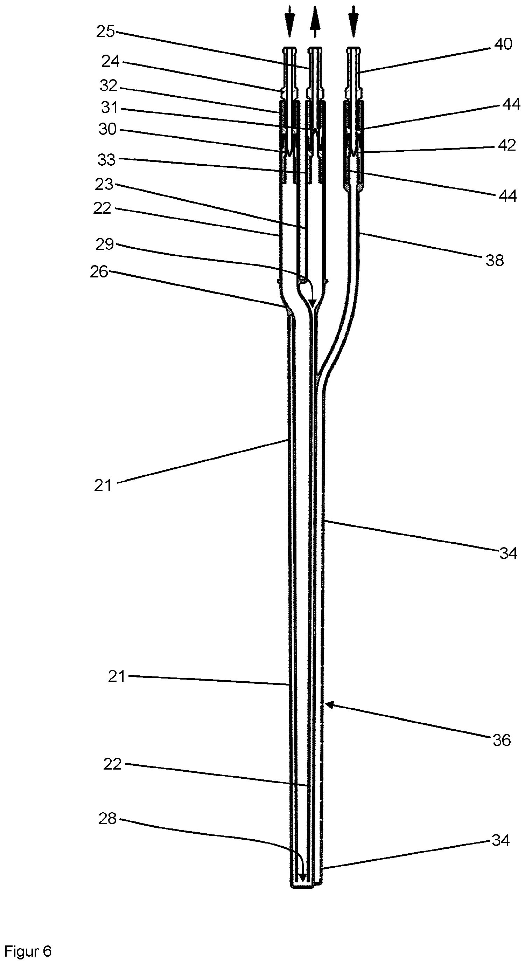

In den Figuren 6 bis 9 sind Abbildungen eines zweiten erfindungsgemäßen medizinischen Implantats gezeigt.

Das zweite erfindungsgemäße medizinische Implantat kann einen Hohlkörper 21 aus einem elastisch oder plastisch verformbaren Kunststoff aufweisen oder einen Hohlkörper 21 aufweisen, dessen Wandungen zumindest bereichsweise aus einem Kunststoff bestehen. Der Hohlkörper 21 kann ein einseitig geschlossenes Zylinderrohr sein. Der Hohlkörper 21 kann beispielsweise aus einem biokompatiblen Kunststoff bestehen. Der Hohlkörper 21 kann für Flüssigkeiten undurchlässig sein. Im Inneren des Hohlkörpers 21 kann ein Innenraum angeordnet sein.

Das für den Hohlkörper 21 zumindest bereichsweise verwendete Material kann für molekularen Sauerstoff und für Kohlendioxid durchlässig sein. Der Hohlkörper 21 beziehungsweise das Material, aus dem der Hohlkörper 21 gefertigt ist, kann hierzu einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweisen. Der Permeabilitätskoeffizient wird dabei nach DIN 53380-4 (11/2006) bestimmt.

Zum Einleiten eines Fluids kann ein Innenraum des Hohlkörpers 21 mit einer Fluidzuleitung 22 aus Kunststoff verbunden sein. Zum Ableiten des Fluids aus dem Hohlkörper 21 heraus kann der Innenraum des Hohlkörpers 21 mit einer Fluidableitung 23 aus Kunststoff verbunden sein. Die Fluidzuleitung 22 und die Fluidableitung 23 können zumindest bereichsweise flexibel und beweglich sein. Die Fluidzuleitung 22 kann an ihrer Rückseite (in Figur 5 rechts oben, in den Figuren 6, 7 und 9 oben) einen Anschluss 24 aufweisen, mit dem die Fluidzuleitung 22 an eine Fluidquelle (nicht gezeigt) angeschlossen werden kann. Ebenso kann die Fluidableitung 23 an ihrer Rückseite einen Anschluss 25 aufweisen, mit dem die Fluidableitung 23 an eine Aufnahme oder ein Abfluss für verbrauchtes Fluid angeschlossen werden kann.

Die Fluidzuleitung 22 und die Fluidableitung 23 können in einer Verbindung 26 zusammengeführt sein, in der die Fluidzuleitung 22 koaxial in die Fluidableitung 23 beziehungsweise in den Hohlkörper 21 geführt sein kann. Die Fluidzuleitung 22 kann koaxial in dem Hohlkörper 21 angeordnet sein. Die Fluidzuleitung 22 kann in dem Hohlkörper 21 bis fast an das vordere geschlossene Ende des Hohlkörpers 21 geführt sein und dort durch eine Einströmöffnung 28 in den Hohlkörper 21 münden. Die Fluidzuleitung 22 kann über die Einströmöffnung 28 in den vorderen Teil des Innenraums des Hohlkörpers 21 münden. Die Fluidableitung 23 kann über eine Ausströmöffnung 29 an der gegenüberliegenden Rückseite des Innenraums des Hohlkörpers 21 mit dem Innenraum des Hohlkörpers 21 verbunden sein. Hierdurch wird sichergestellt, dass das Fluid entlang der Oberfläche der Wandung des gesamten Hohlkörpers 21 strömen kann und dadurch ein Gasaustausch von Sauerstoff und Kohlendioxid durch die Wandung auf der gesamten Länge des Hohlkörpers 21 erfolgen kann. Die Ausströmöffnung 29 kann durch die Verbindung 26 begrenzt sein.

In der Fluidzuleitung 22 kann ein Ventil 30 in Form eines Lippenventils angeordnet sein, das eine Strömung des Fluids in Richtung des Hohlkörpers 21 ermöglicht aber eine Strömung des Fluids in der Richtung vom Hohlkörper 21 weg verhindert. Das Ventil 30 wirkt dann als Einwegventil. In der Fluidableitung 23 kann ein Ventil 31 in Form eines Lippenventils angeordnet sein, das eine Strömung des Fluids in Richtung des Hohlkörpers 21 verhindert aber eine Strömung des Fluids in der Richtung vom Hohlkörper 21 weg erlaubt. Das Ventil 31 wirkt dann als Einwegventil. Das Ventil 31 in der Fluidableitung 23 kann dazu ausgelegt sein, dass es ab einem Mindestdruck des Fluids öffnet. Bevorzugt ist der Mindestdruck am Ventil 31 in der Fluidableitung 23 einstellbar. Der Mindestdruck kann dabei so gewählt werden, dass der Druck des Fluids dazu ausreicht, den Hohlkörper 21 in eine gewünschte äußere Form zu bringen.

Die Ventile 30, 31 können über Ventilgehäuse 32, 33 mit der Fluidzuleitung 22 und der Fluidableitung 23 verbunden sein. Hierzu kann die Fluidzuleitung 22 auf das Ventilgehäuse 32 gesteckt sein und gegebenenfalls zusätzlich befestigt sein. Ebenso kann die Fluidableitung 23 auf Ventilgehäuse 33 gesteckt sein und dort gegebenenfalls zusätzlich befestigt sein.

Der Anschluss 24 kann an seiner Rückseite die Form eines Luer-Lock-Adapters haben. Ebenso kann der Anschluss 25 an seiner Rückseite die Form eines Luer-Lock-Adapters haben. Durch die Anschlüsse 24, 25 kann das Fluid eingespeist und abgeleitet werden. Die Anschlüsse 24, 25 können in die Ventilgehäuse 32, 33 eingeschraubt sein.

Das Ventilgehäuse 32 kann zur Fixierung des Ventils 30 zweiteilig aufgebaut sein. Das Ventilgehäuse 32 kann über ein Innengewinde mit einem Außengewinde des Anschlusses 24 verbunden sein. Das Ventilgehäuse 33 kann zur Fixierung des Ventils 31 zweiteilig aufgebaut sein. Das Ventilgehäuse 33 kann über ein Innengewinde mit einem Außengewinde des Anschlusses 25 verbunden sein. Alle Verbindungen können gasdicht und Druckdicht ausgeführt sein.

Die Fluidzuleitung 22 kann auf das Ventilgehäuse 32 aufgesteckt sein. Es kann vorgesehen sein, dass die Fluidzuleitung 22 dort mit einer Krimphülse (nicht gezeigt) druckdicht und gasdicht befestigt wird. Die Fluidableitung 23 kann auf das Ventilgehäuse 33 aufgesteckt sein. Es kann vorgesehen sein, dass die Fluidableitung 23 dort mit einer Krimphülse (nicht gezeigt) druckdicht und gasdicht befestigt wird.

Der Hohlkörper 21 kann in einen Hohlraum eingebracht werden. Der Hohlkörper 21, beziehungsweise das medizinische Implantat, kann so den Hohlraum mechanisch stützen und stabilisieren. Wenn das Implantat nicht mehr benötigt wird, kann der Hohlkörper 21 durch Anlegen eines Unterdrucks komprimiert werden, beispielsweise indem er evakuiert wird. Der Hohlkörper 21 kann dann wieder aus dem Hohlraum entfernt werden. Insbesondere wenn der Hohlraum eine Kavität in einem Knochen ist, kann dieser Knochendefekt so schonend behandelt werden. Alternativ kann der Hohlkörper 21 auch innerhalb des Körpers abgebaut werden, wenn er aus einem biodegradierbaren Material gefertigt ist.

Im Betrieb kann das Fluid durch den Anschluss 24 in das medizinische Implantat eingespeist werden (so wie das in den Figuren 5, 6, 7 und 9 durch den in den Anschluss 24 hineinweisenden spitzen Pfeil angedeutet ist). Das Fluid kann durch die Fluidzuleitung 22 strömen und bei ausreichendem Druck das Ventil 30 öffnen. Dann kann das Fluid durch die Einströmöffnung 28 in den Hohlkörper 21 und durch den Hohlkörper 21 fließen. Das Fluid kann durch die Fluidableitung 23 bis zum zunächst geschlossenen Ventil 31 strömen. Dabei kann sich im Inneren des Hohlkörpers 21 ein Druck aufbauen. Sobald der Druck am Ventil 31 in der Fluidableitung 23 ausreicht, öffnet sich das Ventil 31 und das Fluid kann durch die Fluidableitung 23 und den Anschluss 25 abfließen (so wie das in den Figuren 5, 6, 7 und 9 durch den vom Anschluss 25 wegweisenden spitzen Pfeil angedeutet ist).

In dem Fluid ist Sauerstoff enthalten. Das Fluid kann durch die Wandung des Hohlkörpers 21 Sauerstoff an die Umgebung des Hohlkörpers 21 abgeben. Gleichzeitig kann das strömende Fluid Kohlendioxid aus der Umgebung des Hohlkörpers 21, das durch die Wandung des Hohlkörpers 21 in den Innenraum diffundiert, aufnehmen, und aus dem medizinischen Implantat durch den Anschluss 25 ableiten. Hierdurch kann die Umgebung des Hohlkörpers 21 mit Sauerstoff versorgt werden und durch die Aufnahme von Kohlendioxid eine Übersäuerung der Umgebung des Hohlkörpers 21 verhindert werden.

In der Fluidzuleitung 22 und/oder in der Fluidableitung 23 können keimundurchlässige aber für das Fluid durchlässige Sterilfilter (nicht gezeigt) angeordnet sein. Insbesondere wenn das Fluid gasförmig ist, kann diese Maßnahme problemlos eingesetzt werden. Wenn das Fluid flüssig ist, muss darauf geachtet werden, dass die Sterilfilter den Fluss des Fluids nicht zu stark hemmen. Das Fluid kann mit dem Sterilfilter von Keimen befreit werden, die ansonsten in den Hohlkörper 21 gelangen könnten und/oder die vom Hohlkörper 21 durch den Anschluss 25 abgeleitet werden könnten. Hierdurch wird die Gefahr einer Infektion des behandelten Patienten und des behandelnden Personals reduziert. Bevorzugt kann der Sterilfilter in der Fluidzuleitung 22 oder der Fluidableitung 23 in Strömungsrichtung hinter dem Ventil 30 oder dem Ventil 31 angeordnet sein oder die Sterilfilter in der Fluidzuleitung 22 und in der Fluidableitung 23 hinter den Ventilen 30, 31 angeordnet sein. Es sind auch andere Verfahren und Möglichkeiten zur Sterilisierung des Fluids möglich. Beispielsweise kann das Fluid mit einer Strahlung sterilisiert werden.

Es kann auch vorgesehen sein, dass der Hohlkörper 21 sowie die angrenzenden Bereiche der Fluidzuleitung 22 und der Fluidableitung 23 mit einer antiseptischen Substanz beschichtet sind oder eine lösbare antiseptische Substanz in dem Material des Hohlkörpers 21 enthalten ist, um eine Infektion zu verhindern.

Zur Behandlung der Umgebung des Hohlkörpers 21 kann zudem vorgesehen sein, dass eine Leitung 34 mit mehreren durchgehenden Öffnungen 36 außen am Hohlkörper 21 befestigt ist. Die Leitung 34 kann mit einer für Flüssigkeiten undurchlässigen Wirkstoffzuleitung 38 verbunden sein. Die Leitung 34 und die Wirkstoffzuleitung 38 können zum Einleiten von Flüssigkeiten zu den Öffnungen 36 vorgesehen sein. Die Wirkstoffzuleitung 38 kann über einen Anschluss 40 mit einer Quelle für pharmazeutische Wirkstofflösungen verbunden sein. Dadurch kann eine pharmazeutische Wirkstofflösung an der Oberfläche der Leitung 34 beziehungsweise des Hohlkörpers 21 abgegeben werden.

Um einen Rückfluss von Flüssigkeiten aus der Leitung 34 zu verhindern, kann ein Ventil 42 zwischen der Wirkstoffzuleitung 38 und dem Anschluss 40 angeordnet sein. Das Ventil 42 ist vorzugsweise ein Einwegventil, wie beispielsweise ein Lippenventil. Das Ventil 42 kann über ein Ventilgehäuse 44 mit der Wirkstoffzuleitung 38 verbunden sein. Hierzu kann die Wirkstoffzuleitung 38 auf das Ventilgehäuse 44 gesteckt sein und gegebenenfalls zusätzlich befestigt sein. Der Anschluss 40 kann an seiner Rückseite die Form eines Luer-Lock-Adapters haben. Das Ventilgehäuse 44 kann zur Fixierung des Ventils 42 zweiteilig aufgebaut sein. Das Ventilgehäuse 44 kann über ein Innengewinde mit einem Außengewinde des Anschlusses 40 verbunden sein. Die Wirkstoffzuleitung 38 kann auf das Ventilgehäuse 44 aufgesteckt sein.

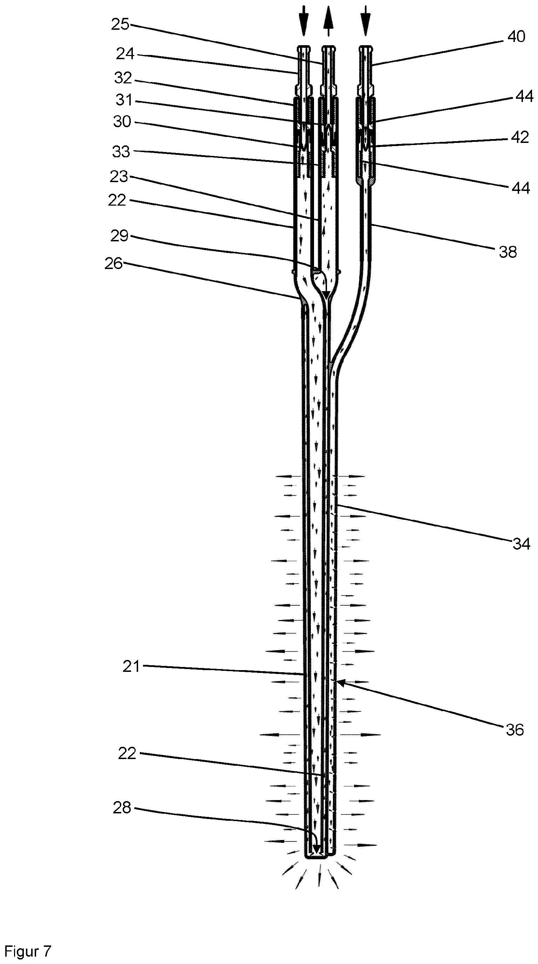

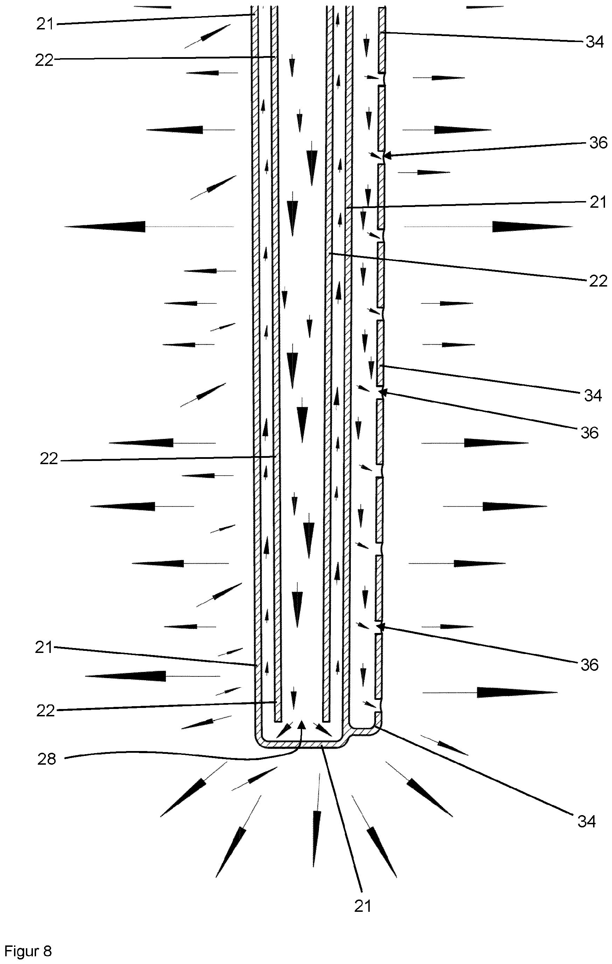

Die spitzen Pfeile in den Figuren 7 und 8 im Inneren des Hohlraums 21, der Fluidzuleitung 22 und der Fluidableitung 23 deuten die Fließrichtung des Fluids während des Betriebs an. Ebenso deuten die spitzen Pfeile in der Leitung 34 den Fluss und die Abgabe eines flüssigen medizinischen Wirkstoffs an. Des Weiteren deuten die spitzen Pfeile im Bereich um den Hohlraum 21 die Abgabe von Sauerstoff aus dem Fluid an (Figuren 7 und 8) und einige kleinere spitze Pfeile die Aufnahme von Kohlendioxid (nur in Figur 8).

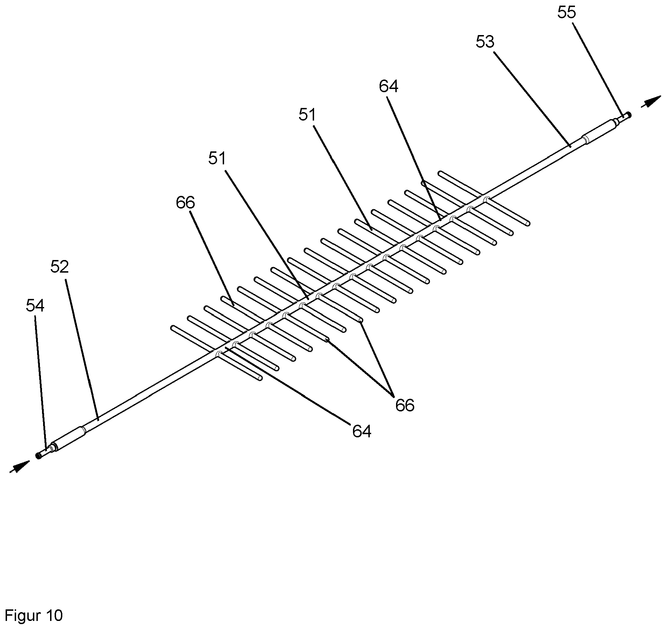

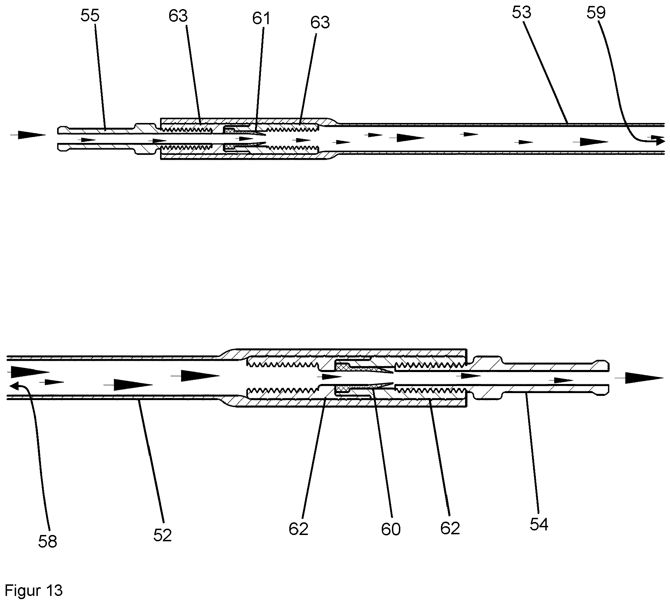

Die Figuren 10 bis 13 zeigen Abbildungen eines dritten Ausführungsbeispiels eines erfindungsgemäßen medizinischen Implantats.

Das dritte erfindungsgemäße medizinische Implantat kann einen Hohlkörper 51 aus einem plastisch verformbaren Kunststoff mit eingearbeiteten Metalldrähten oder einer eingearbeiteten Metallgittermatrix aufweisen. Der Hohlkörper 51 kann einen Hauptstrang 64 und mehreren jeweils paarweise gegenüberliegende und sich senkrecht vom Hauptstrang 64 weg erstreckende Äste 66 aufweisen. Die seitlichen Äste 66 können in etwa 2 mm vom Hauptstrang 64 abstehen.

Der Hohlkörper 51 kann beispielsweise aus einem biokompatiblen Kunststoff bestehen. Der Hohlkörper 51 kann für Flüssigkeiten undurchlässig sein. Im Inneren des Hohlkörpers 51 und damit auch im Inneren des Hauptstrangs 64 und der Äste 66 kann in dem Hohlkörper 51 ein Innenraum angeordnet sein.

Das für den Hohlkörper 51 zumindest bereichsweise verwendete Material kann für molekularen Sauerstoff und für Kohlendioxid durchlässig sein. Der Hohlkörper 51 beziehungsweise das Material, aus dem der Hohlkörper 51 gefertigt ist, kann hierzu einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweisen. Der Permeabilitätskoeffizient wird dabei nach DIN 53380-4 (11/2006) bestimmt.

Zum Einleiten eines Fluids kann ein Innenraum des Hohlkörpers 51 mit einer Fluidzuleitung 52 aus Kunststoff verbunden sein. Zum Ableiten des Fluids aus dem Hohlkörper 51 heraus kann der Innenraum des Hohlkörpers 51 mit einer Fluidableitung 53 aus Kunststoff verbunden sein. Die Fluidzuleitung 52 und die Fluidableitung 53 können zumindest bereichsweise flexibel und beweglich sein. Die Fluidzuleitung 52 kann einen Anschluss 54 aufweisen, mit dem die Fluidzuleitung 52 an eine Fluidquelle (nicht gezeigt) angeschlossen werden kann. Ebenso kann die Fluidableitung 53 einen Anschluss 55 aufweisen, mit dem die Fluidableitung 53 an eine Aufnahme oder ein Abfluss für verbrauchtes Fluid angeschlossen werden kann.

Die Fluidzuleitung 52 und die Fluidableitung 53 können an gegenüberliegenden Seiten des Hohlkörpers 51 in den Hohlkörper 51 beziehungsweise den Hauptstrang 64 einmünden. Die Fluidzuleitung 52 kann über die Einströmöffnung 58 in den Innenraum des Hohlkörpers 51 münden. Die Fluidableitung 53 kann über eine Ausströmöffnung 59 an der gegenüberliegenden Seite des Innenraums des Hohlkörpers 51 mit dem Innenraum des Hohlkörpers 51 verbunden sein. Hierdurch wird sichergestellt, dass das Fluid entlang der Oberfläche der Wandung des gesamten Hohlkörpers 51 strömen kann und dadurch ein Gasaustausch von Sauerstoff und Kohlendioxid durch die Wandung auf der gesamten Länge des Hohlkörpers 51 und auch in dessen Ästen 66 erfolgen kann.

In der Fluidzuleitung 52 kann ein Ventil 60 in Form eines Lippenventils angeordnet sein, das eine Strömung des Fluids in Richtung des Hohlkörpers 51 ermöglicht aber eine Strömung des Fluids in der Richtung vom Hohlkörper 51 weg verhindert. Das Ventil 60 wirkt dann als Einwegventil. In der Fluidableitung 53 kann ein Ventil 61 in Form eines Lippenventils angeordnet sein, das eine Strömung des Fluids in Richtung des Hohlkörpers 51 verhindert aber eine Strömung des Fluids in der Richtung vom Hohlkörper 51 weg erlaubt. Das Ventil 61 wirkt dann als Einwegventil. Das Ventil 61 in der Fluidableitung 53 kann dazu ausgelegt sein, dass es ab einem Mindestdruck des Fluids öffnet. Bevorzugt ist der Mindestdruck am Ventil 61 in der Fluidableitung 53 einstellbar. Der Mindestdruck kann dabei so eingestellt werden, dass der Druck des Fluids dazu ausreicht, den Hohlkörper 51 in eine gewünschte äußere Form zu bringen.

Die Ventile 60, 61 können über Ventilgehäuse 62, 63 mit der Fluidzuleitung 52 und der Fluidableitung 53 verbunden sein. Hierzu kann die Fluidzuleitung 52 auf das Ventilgehäuse 62 gesteckt sein und gegebenenfalls zusätzlich befestigt sein. Ebenso kann die Fluidableitung 53 auf Ventilgehäuse 63 gesteckt sein und dort gegebenenfalls zusätzlich befestigt sein.

Der Anschluss 54 kann an seiner Rückseite die Form eines Luer-Lock-Adapters haben. Ebenso kann der Anschluss 55 an seiner Rückseite die Form eines Luer-Lock-Adapters haben. Durch die Anschlüsse 54, 55 kann das Fluid eingespeist und abgeleitet werden. Die Anschlüsse 54, 55 können in die Ventilgehäuse 62, 63 eingeschraubt sein.

Das Ventilgehäuse 62 kann zur Fixierung des Ventils 60 zweiteilig aufgebaut sein. Das Ventilgehäuse 62 kann über ein Innengewinde mit einem Außengewinde des Anschlusses 54 verbunden sein. Das Ventilgehäuse 63 kann zur Fixierung des Ventils 61 zweiteilig aufgebaut sein. Das Ventilgehäuse 63 kann über ein Innengewinde mit einem Außengewinde des Anschlusses 55 verbunden sein. Alle Verbindungen können gasdicht und Druckdicht ausgeführt sein.

Die Fluidzuleitung 52 kann auf das Ventilgehäuse 62 aufgesteckt sein. Es kann vorgesehen sein, dass die Fluidzuleitung 52 dort mit einer Krimphülse (nicht gezeigt) druckdicht und gasdicht befestigt wird. Die Fluidableitung 53 kann auf das Ventilgehäuse 13 aufgesteckt sein. Es kann vorgesehen sein, dass die Fluidableitung 53 dort mit einer Krimphülse (nicht gezeigt) druckdicht und gasdicht befestigt wird.

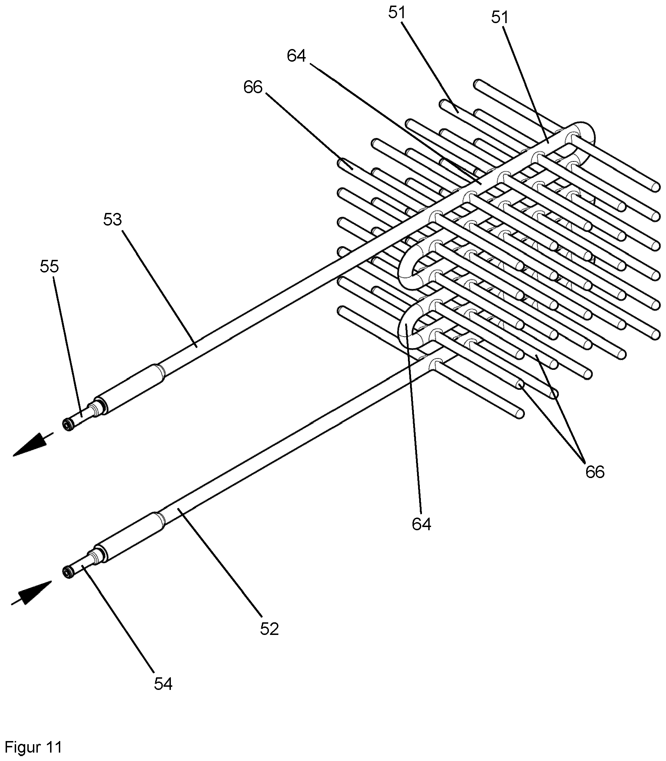

Der Hohlkörper 51 kann gefaltet werden, um eine dreidimensionale Struktur zu erzeugen (siehe Figur 11). Ferner kann der Hohlkörper 51 zur Versorgung und zum Wachstum von Zellkulturen von Knochenzellen verwendet werden, die auf der Oberfläche des Hohlkörpers 51 angeordnet sind. Anschließend kann der so präparierte Hohlkörper 51 in einen Hohlraum eingebracht werden. Der Hohlkörper 51, beziehungsweise das medizinische Implantat, kann so den Hohlraum mechanisch stützen und stabilisieren. Wenn das medizinische Implantat nicht mehr benötigt wird, kann der Hohlkörper 51 durch Anlegen eines Unterdrucks komprimiert werden, beispielsweise indem er evakuiert wird. Der Hohlkörper 51 kann dann wieder bequem aus dem Hohlraum entfernt werden. Insbesondere wenn der Hohlraum eine Kavität in einem Knochen ist, kann dieser Knochendefekt so schonend behandelt werden.

Alternativ kann der Hohlkörper 51 auch innerhalb des Körpers abgebaut werden, wenn er aus einem biodegradierbaren Material gefertigt ist.

Im Betrieb kann das Fluid durch den Anschluss 54 in das medizinische Implantat eingespeist werden (so wie das in den Figuren 10 bis 13 durch den in den Anschluss 54 hineinweisenden spitzen Pfeil angedeutet ist). Das Fluid kann durch die Fluidzuleitung 52 strömen und bei ausreichendem Druck das Ventil 60 öffnen. Dann kann das Fluid durch die Einströmöffnung 58 in den Hohlkörper 51 und durch den Hauptstrang 64 und die Äste 66 des Hohlkörpers 51 fließen. Das Fluid kann durch die Fluidableitung 53 bis zum zunächst geschlossenen Ventil 61 strömen. Dabei kann sich im Inneren des Hohlkörpers 51 ein Druck aufbauen. Sobald der Druck am Ventil 61 in der Fluidableitung 53 ausreicht, öffnet sich das Ventil 61 und das Fluid kann durch die Fluidableitung 53 und den Anschluss 55 abfließen (so wie das in den Figuren 10 bis 13 durch den vom Anschluss 55 wegweisenden spitzen Pfeil angedeutet ist).

In dem Fluid ist Sauerstoff enthalten. Das Fluid kann durch die Wandung des Hohlkörpers 51 Sauerstoff an die Umgebung des Hohlkörpers 51 abgeben. Gleichzeitig kann das strömende Fluid Kohlendioxid aus der Umgebung des Hohlkörpers 51, das durch die Wandung des Hohlkörpers 51 in den Innenraum diffundiert, aufnehmen, und aus dem medizinischen Implantat durch den Anschluss 55 ableiten. Hierdurch kann die Umgebung des Hohlkörpers 51 mit Sauerstoff versorgt werden und durch die Aufnahme von Kohlendioxid eine Übersäuerung der Umgebung des Hohlkörpers 51 verhindert werden.

In der Fluidzuleitung 52 und/oder in der Fluidableitung 53 können keimundurchlässige aber für das Fluid durchlässige Sterilfilter (nicht gezeigt) angeordnet sein. Insbesondere wenn das Fluid gasförmig ist, kann diese Maßnahme problemlos eingesetzt werden. Wenn das Fluid flüssig ist, muss darauf geachtet werden, dass die Sterilfilter den Fluss des Fluids nicht zu stark hemmen. Das Fluid kann mit dem Sterilfilter von Keimen befreit werden, die ansonsten in den Hohlkörper 51 gelangen könnten und/oder die vom Hohlkörper 51 durch den Anschluss 55 abgeleitet werden könnten. Hierdurch wird die Gefahr einer Infektion des behandelten Patienten und des behandelnden Personals reduziert. Bevorzugt kann der Sterilfilter in der Fluidzuleitung 52 oder der Fluidableitung 53 in Strömungsrichtung hinter dem Ventil 60 oder dem Ventil 61 angeordnet sein oder die Sterilfilter in der Fluidzuleitung 52 und in der Fluidableitung 53 hinter den Ventilen 60, 61 angeordnet sein. Es sind auch andere Verfahren und Möglichkeiten zur Sterilisierung des Fluids möglich. Beispielsweise kann das Fluid mit einer Strahlung sterilisiert werden.

Es kann auch vorgesehen sein, dass der Hohlkörper 51 sowie gegebenenfalls die angrenzenden Bereiche der Fluidzuleitung 52 und der Fluidableitung 53 mit einer antiseptischen Substanz beschichtet sind oder eine lösbare antiseptische Substanz in dem Material des Hohlkörpers 51 enthalten ist, um eine Infektion zu verhindern.

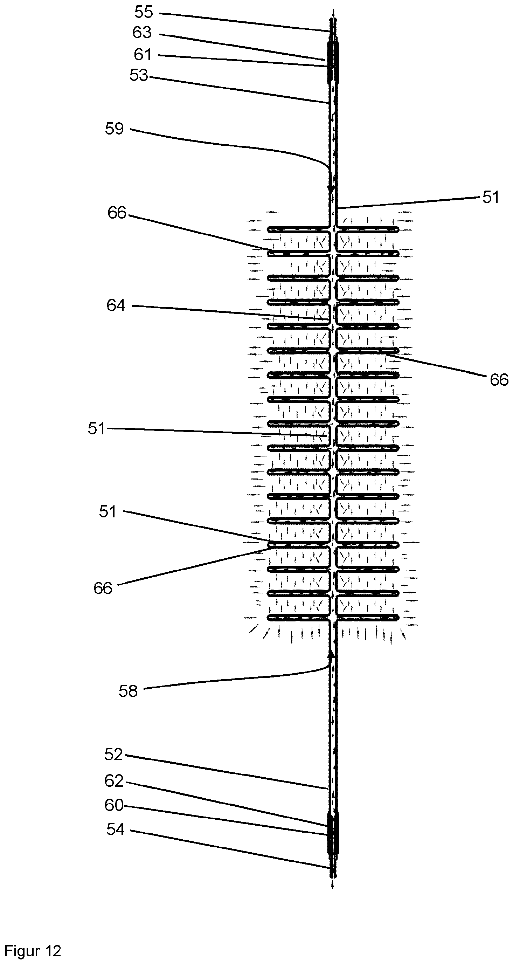

Die spitzen Pfeile in Figur 12 im Inneren des Hohlraums 51, der Fluidzuleitung 52 und der Fluidableitung 53 deuten die Fließrichtung des Fluids während des Betriebs an. Des Weiteren deuten die spitzen Pfeile im Bereich um den Hohlraum 51 die Abgabe von Sauerstoff aus dem Fluid an.

Das medizinische Implantat kann zum Belüften und Vermehren einer Zellkultur für Knochenzellen an der Oberfläche des Hohlkörpers 1, 21, 51 extrakorporal verwendet werden. Mit der gewachsenen Zellkultur versehen kann das medizinische Implantat anschließend implantiert werden und dann noch weiter im Inneren des Körpers belüftet werden, um ein weiteres Vermehren und Anwachse der Zellen im Knochendefekt zu fördern.

### Bezugszeichenliste

- 1, 21, 51: Hohlkörper
- 2, 22, 52: Fluidzuleitung
- 3, 23, 53: Fluidableitung
- 4, 24, 54: Anschluss
- 5, 25, 55: Anschluss
- 6, 26: Verbindung
- 8, 28, 58: Einströmöffnung
- 9, 29, 59: Ausströmöffnung
- 10, 30, 60: Ventil / Lippenventil
- 11, 31, 61: Ventil / Lippenventil
- 12, 32, 62: Ventilgehäuse
- 13, 33, 63: Ventilgehäuse
- 34: Leitung
- 36: Öffnung
- 38: Wirkstoffzuleitung
- 40: Anschluss
- 42: Ventil
- 44: Ventilgehäuse
- 64: Hauptstrang
- 66: Ast

## Patentansprüche

1. Medizinisches Implantat zur Behandlung von Knochendefekten aufweisend mindestens einen Hohlkörper (1, 21, 51), der einen Innenraum im Inneren des Hohlkörpers (1, 21, 51) begrenzt,
eine Fluidzuleitung (2, 22, 52), die mit dem Innenraum des Hohlkörpers (1, 21, 51) fluiddurchlässig verbunden ist,
eine Fluidableitung (3, 23, 53), die mit dem Innenraum des Hohlkörpers (1, 21, 51) fluiddurchlässig verbunden ist,
wobei der Hohlkörper (1, 21, 51) zumindest bereichsweise oder vollständig aus zumindest einem Kunststoff besteht, wobei der zumindest eine Kunststoff für Flüssigkeiten undurchlässig ist und für Sauerstoff und für Kohlendioxid durchlässig ist, so dass Sauerstoff aus einem durch den Hohlkörper (1, 21, 51) geleiteten Fluid an die Umgebung des Hohlkörpers (1, 21, 51) abgebbar ist und Kohlendioxid aus der Umgebung des Hohlkörpers (1, 21, 51) in das Fluid aufnehmbar ist, **dadurch gekennzeichnet, dass**
der Hohlkörper (1, 21, 51) oder der zumindest eine Kunststoff des Hohlkörpers (1, 21, 51) einen Permeabilitätskoeffizienten für Sauerstoff von größer oder gleich 0,5 cm³/(m²*d*bar) und einen Permeabilitätskoeffizienten für Kohlendioxid von größer oder gleich 0,5 cm³/(m²*d*bar) aufweist.

2. Medizinisches Implantat nach Anspruch 1, **dadurch gekennzeichnet, dass** der Hohlkörper (1, 21, 51) oder der zumindest eine Kunststoff des Hohlkörpers (1, 21, 51) einen Permeabilitätskoeffizienten für Sauerstoff von größer oder gleich 1 cm³/(m²*d*bar) und einen Permeabilitätskoeffizienten für Kohlendioxid von größer oder gleich 1 cm³/(m²*d*bar) aufweist.

3. Medizinisches Implantat nach Anspruch 1 oder 2, **dadurch gekennzeichnet, dass** die Fluidzuleitung (2, 22, 52) und die Fluidableitung (3, 23, 53) derart in den Hohlkörper (1, 21, 51) münden, dass bei einem Durchströmen des Fluids aus der Fluidzuleitung (2, 22, 52) durch den Hohlkörper (1, 21, 51) in die Fluidableitung (3, 23, 53) das Fluid über eine gesamte Innenfläche des Hohlkörpers (1, 21, 51) strömt oder zumindest über 50% der gesamten Innenfläche des Hohlkörpers (1, 21, 51) strömt.

4. Medizinisches Implantat nach einem der vorangehenden Ansprüche, **dadurch gekennzeichnet, dass**
eine Einströmöffnung (8, 28, 58) der Fluidzuleitung (2, 22, 52), mit der die Fluidzuleitung (2, 22, 52) in den Innenraum mündet, räumlich getrennt von einer Ausströmöffnung (9, 29, 59) der Fluidableitung (3, 23, 53) angeordnet ist, wobei die Ausströmöffnung (9, 29, 59) die Einmündung des Innenraums in die Fluidableitung (3, 23, 53) bildet, wobei vorzugsweise
die Einströmöffnung (8, 28, 58) der Fluidzuleitung (2, 22, 52) an einem ersten Ende des Hohlkörpers (1, 21, 51) angeordnet ist und die Ausströmöffnung (9, 29, 59) an einem dem ersten Ende gegenüberliegenden zweiten Ende des Hohlkörpers (1, 21, 51) angeordnet ist.

5. Medizinisches Implantat nach einem der vorangehenden Ansprüche, **dadurch gekennzeichnet, dass**
die Fluidzuleitung (2, 22) und die Fluidableitung (3, 23) beide gemeinsam auf einer Seite des Hohlkörpers (1, 21) mit dem Hohlkörper (1, 21) verbunden sind, und/oder
in der Fluidzuleitung (2, 22, 52) und/oder der Fluidableitung (3, 23, 53) ein Ventil (10, 11, 30, 31, 60, 61) angeordnet ist, insbesondere ein Einwegventil angeordnet ist, wobei das Einwegventil vorzugsweise ein Rückschlagventil ist.

6. Medizinisches Implantat nach einem der vorangehenden Ansprüche, **dadurch gekennzeichnet, dass**
die Fluidzuleitung (2, 22, 52) und die Fluidableitung (3, 23, 53) aus einem für Sauerstoff und Kohlendioxid nicht durchlässigen Material bestehen, vorzugsweise aus einem für Sauerstoff und Kohlendioxid nicht durchlässigen Kunststoff bestehen, und/oder
der Hohlkörper (1, 21, 51) oder der zumindest eine Kunststoff des Hohlkörpers (1, 21, 51) mindestens einen antiseptischen Wirkstoff enthält oder mit mindestens einem antiseptischen Wirkstoff beschichtet ist.

7. Medizinisches Implantat nach einem der vorangehenden Ansprüche, **dadurch gekennzeichnet, dass**
der Hohlkörper (1, 21) ein Hohlzylinder ist oder der Hohlkörper (51) einen Hauptstrang (64) in Form eines Hohlzylinders aufweist, wobei die Fluidzuleitung (2, 22, 52) und die Fluidableitung (3, 23, 53) im Bereich gegenüberliegender Grundflächen des Hohlzylinders in den Hohlzylinder münden, wobei vorzugsweise der Hohlzylinder einen Teil der Fluidzuleitung (2, 22) oder der Fluidableitung (3, 23) koaxial umgibt, und/oder der Hohlkörper (1, 21, 51) als perforierter Metallkörper oder Kunststoffkörper ausgeführt ist, dessen Außenseite mit einer für Sauerstoff und für Kohlendioxid permeablen Kunststoffschicht überdeckt ist.

8. Medizinisches Implantat nach einem der vorangehenden Ansprüche, **dadurch gekennzeichnet, dass**
der Hohlkörper (1, 21, 51) einen für Sauerstoff und Kohlendioxid permeablen und plastisch verformbaren Kunststoffmantel aufweist, wobei vorzugsweise der Kunststoffmantel spiralförmige und/oder netzartig angeordnete Metalldrähte enthält, und/oder
am Hohlkörper (21) eine Leitung (34) mit mehreren Öffnungen (36) zur Abgabe eines Wirkstoffs angeordnet ist, wobei die Leitung (34) mit einer Wirkstoffzuleitung (38) verbunden ist, wobei bevorzugt die Summe der freien Querschnittsflächen der Öffnungen (36) kleiner ist als der freie Querschnitt der Leitung (34) und der Wirkstoffzuleitung (38).

9. Medizinisches Implantat nach einem der vorangehenden Ansprüche, **dadurch gekennzeichnet, dass**
der zumindest eine Kunststoff ein elastischer und/oder plastischer, nicht biodegradierbaren Kunststoff ist, wobei bevorzugt der zumindest eine Kunststoff ausgewählt ist aus Polyurethan, Ethylen-Propylen-Dien-Kautschuk EPDM und Silikon, oder
der zumindest eine Kunststoff ein elastischer und/oder plastischer, biodegradierbaren Kunststoff ist, wobei bevorzugt der zumindest eine Kunststoff ausgewählt ist aus Gelatine, vernetztem Kollagen und vernetztem Albumin.

10. Medizinisches Implantat nach einem der vorangehenden Ansprüche, **dadurch gekennzeichnet, dass**
der Hohlkörper (1, 21, 51) durch Änderung des Drucks in seinem Innenraum gegenüber der umgebenden Atmosphäre expandierbar und/oder zusammenziehbar ist, und/oder
der Hohlkörper (51) einen Hauptstrang (64) und mehrere seitlich vom Hauptstrang (64) abgehende Äste (66) aufweist, wobei der Innenraum des Hohlkörpers (51) sich im Hauptstrang (64) und in den Ästen (66) erstreckt und die Fluidzuleitung (52) und die Fluidableitung (53) mit dem Hauptstrang (64) verbunden sind, wobei vorzugsweise zumindest die Äste (66) oder Wandungen der Äste (66) aus dem Kunststoff bestehen oder mit dem Kunststoff aufgebaut sind, und/oder
der Hohlkörper (1, 21, 51) aus zumindest einem resorbierbaren und/oder biodegradierbaren Material besteht.

11. Knochendefektbehandlungssystem aufweisend ein medizinisches Implantat nach einem der vorangehenden Ansprüche und das Fluid, wobei das Fluid Sauerstoff enthält und zur Aufnahme von Kohlendioxid geeignet ist.

12. Knochendefektbehandlungssystem nach Anspruch 11, **dadurch gekennzeichnet, dass** das Fluid ausgewählt ist aus Luft, Sauerstoff, mit Sauerstoff gesättigter Kochsalzlösung, mit Sauerstoff gesättigter Ringer-Lösung, mit Sauerstoff gesättigter Ringer-Lactat-Lösung, mit Sauerstoff gesättigter Phosphatpufferlösung und mit Sauerstoff gesättigtem Perfluordecalin oder einem Gemisch aus zumindest zwei der genannten Gase oder Flüssigkeiten, und/oder
das Knochendefektbehandlungssystem ein Knochenersatzmaterial aufweist, wobei vorzugsweise das Knochenersatzmaterial auf der äußeren Oberfläche des Hohlkörpers aufgetragen ist oder auf die äußere Oberfläche des Hohlkörpers aufbringbar ist.

13. Knochendefektbehandlungssystem nach Anspruch 12, **dadurch gekennzeichnet, dass**
das Knochenersatzmaterial ausgewählt ist aus einem nicht biodegradierbaren, einem teildegradierbaren, einem vollständig biodegradierbaren Knochenersatzmaterial und deren Mischungen, oder
das Knochenersatzmaterial ausgewählt ist aus autologem Knochengewebe, allogenem Knochengewebe, Hydroxylapatit, Carbonatapatit, β-Tricalciumphosphat, α-Tricalciumphosphat, Calcium-Dihydrat, Brushit, Monetit und deren Mischungen, oder das Knochenersatzmaterial lebende Zellen enthält und/oder mit lebenden Zellen auf seiner Oberfläche besiedelt ist.

14. Verfahren zum Begasen einer Oberfläche eines medizinischen Implantats, insbesondere eines medizinischen Implantats nach einem der Ansprüche 1 bis 10, bevorzugt mit einem Knochendefektbehandlungssystem nach einem der Ansprüche 11 bis 13, aufweisend:
A) Einleiten eines Fluids enthaltend Sauerstoff in einen Innenraum eines Hohlkörpers (1, 21, 51) des medizinischen Implantats; **gekennzeichnet durch**:
B) Abgeben von gasförmigem Sauerstoff aus dem Fluid durch einen den Innenraum des Hohlkörpers (1, 21, 51) begrenzenden Kunststoff hindurch an die Umgebung des Hohlkörpers (1, 21, 51);
C) Aufnehmen von gasförmigem Kohlendioxid aus der Umgebung des Hohlkörpers (1, 21, 51) durch den, den Innenraum begrenzenden Kunststoff hindurch in das Fluid;
D) Hindurchleiten des Fluids durch den Innenraum des Hohlkörpers (1, 21, 51) und Ableiten des Fluids aus dem Innenraum des Hohlkörpers (1, 21, 51),
wobei das Verfahren nicht zur medizinischen Behandlung eines menschlichen oder tierischen Körpers durchgeführt wird.

15. Verfahren nach Anspruch 14, **dadurch gekennzeichnet, dass**
vor Schritt A) der Hohlkörper (1, 21, 51) in einen Hohlraum eingeführt wird und ein Knochenersatzmaterial auf die Oberfläche des medizinischen Implantats aufgebracht wird und/oder in den Hohlraum zwischen das medizinische Implantat und den Hohlraum begrenzende Innenwände eingebracht wird, und/oder
das Verfahren zur extrakorporalen Vermehrung von Zellen, insbesondere von Knochenzellen, angewendet wird, wobei vor Schritt A) die Zellen auf der äußeren Oberfläche des Hohlkörpers (1, 21, 51) aufgebracht werden, bevorzugt zusammen mit einer Nährstofflösung und/oder wachstumsfördernden Substanzen auf der äußeren Oberfläche des Hohlkörpers (1, 21, 51) aufgebracht werden.

## Claims

1. A medical implant for treating bone defects, having
at least one hollow body (1, 21, 51), which delimits an inner chamber in the interior of the hollow body (1, 21, 51),
at least one fluid feed line (2, 22, 52), which is connected in fluid-permeable manner with the inner chamber of the hollow body (1, 21, 51),
at least one fluid discharge line (3, 23, 53), which is connected in fluid-permeable manner with the inner chamber of the hollow body (1, 21, 51),
wherein the hollow body (1, 21, 51) consists at least in places or wholly of at least one plastics material, wherein the at least one plastics material is impermeable to liquids and is permeable to oxygen and to carbon dioxide, such that oxygen is deliverable from a fluid passed through the hollow body (1, 21, 51) to the surroundings of the hollow body (1, 21, 51) and carbon dioxide is absorbable from the surroundings of the hollow body (1, 21, 51) into the fluid, **characterized in that**
the hollow body (1, 21, 51) or the at least one plastics material of the hollow body (1, 21, 51) has a permeability coefficient for oxygen of greater than or equal to 0.5 cm³/(m²*d*bar) and a permeability coefficient for carbon dioxide of greater than or equal to 0.5 cm³/(m²*d*bar).

2. The medical implant according to Claim 1, **characterized in that**
the hollow body (1, 21, 51) or the at least one plastics material of the hollow body (1, 21, 51) has a permeability coefficient for oxygen of greater than or equal to 1 cm³/(m²*d*bar) and a permeability coefficient for carbon dioxide of greater than or equal to 1 cm³/(m²*d*bar).

3. The medical implant according to Claim 1 or 2, **characterized in that**
the fluid feed line (2, 22, 52) and the fluid discharge line (3, 23, 53) lead in such a way into the hollow body (1, 21, 51) that, when the fluid from the fluid feed line (2, 22, 52) flows through the hollow body (1, 21, 51) into the fluid discharge line (3, 23, 53), the fluid flows over an entire inner surface of the hollow body (1, 21, 51) or at least over 50% of the entire inner surface of the hollow body (1, 21, 51).

4. The medical implant according to any one of the preceding claims, **characterized in that** an inflow opening (8, 28, 58) of the fluid feed line (2, 22, 52), with which the fluid feed line (2, 22, 52) leads into the inner chamber, is arranged spatially separately from an outflow opening (9, 29, 59) of the fluid discharge line (3, 23, 53), wherein the outflow opening (9, 29, 59) forms the point where the inner chamber opens into the fluid discharge line (3, 23, 53), wherein preferably
the inflow opening (8, 28, 58) of the fluid feed line (2, 22, 52) is arranged at a first end of the hollow body (1, 21, 51) and the outflow opening (9, 29, 59) is arranged at a second end of the hollow body (1, 21, 51) opposite the first end.

5. The medical implant according to any one of the preceding claims, **characterized in that** the fluid feed line (2, 22) and the fluid discharge line (3, 23) are both jointly connected with the hollow body (1, 21) on one side of the hollow body (1, 21), and/or
a valve (10, 11, 30, 31, 60, 61) or a one-way valve or a non-return valve is arranged in the fluid feed line (2, 22, 52) and/or the fluid discharge line (3, 23, 53).

6. The medical implant according to any one of the preceding claims, **characterized in that** the fluid feed line (2, 22, 52) and the fluid discharge line (3, 23, 53) consist of a material which is not permeable to oxygen and carbon dioxide or consist of a plastics material which is not permeable to oxygen and carbon dioxide, and/or
the hollow body (1, 21, 51) or the at least one plastics material of the hollow body (1, 21, 51) contains at least one antiseptic active ingredient or is coated with at least one antiseptic active ingredient.

7. The medical implant according to any one of the preceding claims, **characterized in that** the hollow body (1, 21) is a hollow cylinder or the hollow body (51) has a main part (64) in the form of a hollow cylinder, wherein the fluid feed line (2, 22, 52) and the fluid discharge line (3, 23, 53) lead into the hollow cylinder in the region of opposing base faces of the hollow cylinder, wherein the hollow cylinder preferably coaxially surrounds a part of the fluid feed line (2, 22) or of the fluid discharge line (3, 23), and/or
the hollow body (1, 21, 51) is embodied as a perforated metal body or plastics material body, the outside of which is covered with a plastics layer permeable to oxygen and to carbon dioxide.

8. The medical implant according to any one of the preceding claims, **characterized in that** the hollow body (1, 21, 51) has a plastically deformable plastics jacket permeable to oxygen and carbon dioxide, wherein the plastics jacket preferably contains spiral metal wires and/or metal wires arranged in reticular manner, and/or
a line (34) with a plurality of openings (36) for delivering an active ingredient is arranged on the hollow body (21), wherein the line (34) is connected with an active ingredient feed line (38), wherein preferably the sum of the free cross-sectional areas of the openings (36) is less than the free cross-section of the line (34) and of the active ingredient feed line (38).

9. The medical implant according to any one of the preceding claims, **characterized in that** the at least one plastics material is an elastic and/or plastic, non-biodegradable plastics material, wherein the at least one plastics material is preferably selected from polyurethane, ethylene-propylene-diene rubber EPDM and silicone, or
the at least one plastics material is an elastic and/or plastic biodegradable plastics material, wherein the at least one plastics material is preferably selected from gelatin, crosslinked collagen and crosslinked albumin.

10. The medical implant according to any one of the preceding claims, **characterized in that** the hollow body (1, 21, 51) is expandable and/or contractable by changing the pressure in the inner chamber thereof relative to the surrounding atmosphere, and/or
the hollow body (51) has a main part (64) and a plurality of branches (66) extending laterally from the main part (64), wherein the inner chamber of the hollow body (51) extends in the main part (64) and in the branches (66) and the fluid feed line (52) and the fluid discharge line (53) are connected with the main part (64), wherein preferably at least the branches (66) or walls of the branches (66) consist of the plastics material or are constructed using the plastics material, and/or
the hollow body (1, 21, 51) consists of at least one absorbable and/or biodegradable material.

11. A bone defect treatment system having a medical implant according to any one of the preceding claims and the fluid, wherein the fluid contains oxygen and is suitable for absorbing carbon dioxide.

12. The bone defect treatment system according to Claim 11, **characterized in that**
the fluid is selected from air, oxygen, oxygen-saturated saline, oxygen-saturated Ringer's solution, oxygen-saturated Ringer's lactate solution, oxygen-saturated phosphate buffer solution and oxygen-saturated perfluorodecalin or a mixture of at least two of the stated gases or liquids, and/or
the bone defect treatment system includes a bone substitute material, wherein preferably the bone substitute material has been applied to the external surface of the hollow body or may be applied to the external surface of the hollow body.

13. The bone defect treatment system according to Claim 12, **characterized in that** the bone substitute material is selected from a non-biodegradable, a partially degradable or a fully biodegradable bone substitute material and mixtures thereof, or
the bone substitute material is selected from autologous bone tissue, allogeneic bone tissue, hydroxyapatite, carbonate apatite, β-tricalcium phosphate, α-tricalcium phosphate, calcium dihydrate, brushite, monetite and mixtures thereof, or
the bone substitute material contains living cells and/or is colonized with living cells on the surface thereof.

14. A method for gas-flushing a surface of a medical implant, in particular of a medical implant according to any one of Claims 1 to 10, preferably with a bone defect treatment system according to any one of Claims 11 to 13, comprising:
A) Feeding a fluid containing oxygen into an inner chamber of a hollow body (1, 21, 51) of the medical implant; **characterized by**:
B) Delivering oxygen from the fluid through a plastics material delimiting the inner chamber of the hollow body (1, 21, 51) to the surroundings of the hollow body (1, 21, 51);
C) Absorbing gaseous carbon dioxide from the surroundings of the hollow body (1, 21, 51) through the plastics material delimiting the inner chamber into the fluid;
D) Passing the fluid through the inner chamber of the hollow body (1, 21, 51) and discharging the fluid from the inner chamber of the hollow body (1, 21, 51),
wherein the method is not performed for medical treatment of a human or animal body.

15. The method according to Claim 14, **characterized in that**
the hollow body (1, 21, 51) is introduced into a cavity prior to step A) and a bone substitute material is applied to the surface of the medical implant and/or introduced into the cavity between the medical implant and the inner walls delimiting the cavity, and/or
the method is applied to the extracorporeal multiplication of cells, in particular of bone cells, wherein prior to step A) the cells are applied to the external surface of the hollow body (1, 21, 51), preferably are applied together with a nutrient solution and/or growth promoting substances to the external surface of the hollow body (1, 21, 51).

## Revendications

1. Implant médical pour le traitement de défauts osseux présentant au moins un corps creux (1, 21, 51) qui délimite un espace intérieur à l'intérieur du corps creux (1, 21, 51), une conduite d'amenée de fluide (2, 22, 52) reliée à l'espace intérieur du corps creux (1, 21, 51) de façon perméable aux fluides,
une conduite d'évacuation de fluide (3, 23, 53) reliée à l'espace intérieur du corps creux (1, 21, 51) de façon perméable aux fluides,
dans lequel le corps creux (1, 21, 51) est au moins partiellement ou totalement constitué d'au moins une matière plastique, dans lequel l'au moins une matière plastique est imperméable aux liquides et perméable à l'oxygène et au dioxyde de carbone, de sorte que de l'oxygène peut être distribué dans l'environnement du corps creux (1, 21, 51) à partir d'un fluide transporté à travers le corps creux (1, 21, 51) et que du dioxyde de carbone peut être reçu dans le fluide à partir de l'environnement du corps creux (1, 21, 51), **caractérisé en ce que** le corps creux (1, 21, 51) ou l'au moins une matière plastique du corps creux (1, 21, 51) présente un coefficient de perméabilité à l'oxygène supérieur ou égal à 0,5 cm³/(m²*d*bar) et un coefficient de perméabilité au dioxyde de carbone supérieur ou égal à 0,5 cm³/(m²*d*bar).

2. Implant médical selon la revendication 1, **caractérisé en ce que**
le corps creux (1, 21, 51) ou l'au moins une matière plastique du corps creux (1, 21, 51) présente un coefficient de perméabilité à l'oxygène supérieur ou égal à 1 cm³/(m²*d*bar) et un coefficient de perméabilité au dioxyde de carbone supérieur ou égal à 1 cm³/(m²*d*bar).

3. Implant médical selon la revendication 1 ou 2, **caractérisé en ce que**
la conduite d'amenée de fluide (2, 22, 52) et la conduite d'évacuation de fluide (3, 23, 53) débouchent dans le corps creux (1, 21, 51) de telle sorte que, lors d'un écoulement du fluide de la conduite d'amenée de fluide (2, 22, 52) à la conduite d'évacuation de fluide (3, 23, 53) en passant par le corps creux (1, 21, 51), le fluide s'écoule à travers toute une surface intérieure du corps creux (1, 21, 51) ou s'écoule à travers au moins 50 % de toute la surface intérieure du corps creux (1, 21, 51).

4. Implant médical selon l'une des revendications précédentes, **caractérisé en ce que** une ouverture d'admission (8, 28, 58) de la conduite d'amenée de fluide (2, 22, 52), avec laquelle la conduite d'amenée de fluide (2, 22, 52) débouche dans l'espace intérieur, est disposée de manière à être spatialement séparée d'une ouverture de sortie (9, 29, 59) de la conduite d'évacuation de fluide (3, 23, 53), dans lequel l'ouverture de sortie (9, 29, 59) forme l'embouchure de l'espace intérieur dans la conduite d'évacuation de fluide (3, 23, 53), dans lequel de préférence
l'ouverture d'admission (8, 28, 58) de la conduite d'amenée de fluide (2, 22, 52) est disposée au niveau d'une première extrémité du corps creux (1, 21, 51) et l'ouverture de sortie (9, 29, 59) est disposée au niveau d'une seconde extrémité du corps creux (1, 21, 51) opposée à la première extrémité.

5. Implant médical selon l'une des revendications précédentes, **caractérisé en ce que**
la conduite d'amenée de fluide (2, 22) et la conduite d'évacuation de fluide (3, 23) sont conjointement reliées au corps creux (1, 21) sur un côté du corps creux (1, 21), et/ou une soupape (10, 11, 30, 31, 60, 61), en particulier une soupape unidirectionnelle, est disposée dans la conduite d'amenée de fluide (2, 22, 52) et/ou dans la conduite d'évacuation de fluide (3, 23, 53), dans lequel la soupape unidirectionnelle est de préférence une soupape antiretour.

6. Implant médical selon l'une des revendications précédentes, **caractérisé en ce que**
la conduite d'amenée de fluide (2, 22, 52) et la conduite d'évacuation de fluide (3, 23, 53) sont constituées d'un matériau imperméable à l'oxygène et au dioxyde de carbone, de préférence d'une matière plastique imperméable à l'oxygène et au dioxyde de carbone, et/ou le corps creux (1, 21, 51) ou l'au moins une matière plastique du corps creux (1, 21, 51) contient au moins un principe actif antiseptique ou est revêtu d'au moins un principe actif antiseptique.

7. Implant médical selon l'une des revendications précédentes, **caractérisé en ce que**
le corps creux (1, 21) est un cylindre creux ou le corps creux (51) présente une branche principale (64) sous la forme d'un cylindre creux, dans lequel la conduite d'amenée de fluide (2, 22, 52) et la conduite d'évacuation de fluide (3, 23, 53) débouchent dans le cylindre creux dans la zone de surfaces de base opposées du cylindre creux, dans lequel de préférence le cylindre creux entoure de manière coaxiale une partie de la conduite d'amenée de fluide (2, 22) ou de la conduite d'évacuation de fluide (3, 23), et/ou le corps creux (1, 21, 51) est réalisé comme un corps métallique ou un corps en matière plastique perforé dont le côté extérieur est recouvert d'une couche de matière plastique perméable à l'oxygène et au dioxyde de carbone.

8. Implant médical selon l'une des revendications précédentes, **caractérisé en ce que**
le corps creux (1, 21, 51) présente une enveloppe en matière plastique perméable à l'oxygène et au dioxyde de carbone et plastiquement déformable, dans lequel de préférence l'enveloppe en matière plastique contient des fils métalliques en forme de spirale et/ou disposés à la manière d'un réseau, et/ou
une conduite (34) comportant plusieurs ouvertures (36) pour la distribution d'un principe actif est disposée sur le corps creux (21), dans lequel la conduite (34) est reliée à une conduite d'amenée de principe actif (38), dans lequel de préférence la somme des surfaces de section transversale libres des ouvertures (36) est inférieure à la section transversale libre de la conduite (34) et de la conduite d'amenée de principe actif (38).

9. Implant médical selon l'une des revendications précédentes, **caractérisé en ce que**
l'au moins une matière plastique est une matière plastique non biodégradable élastique et/ou plastique, dans lequel de préférence l'au moins une matière plastique est choisie parmi polyuréthane, caoutchouc éthylène-propylène-diène (EPDM) et silicone, ou
l'au moins une matière plastique est une matière plastique biodégradable élastique et/ou plastique, dans lequel de préférence l'au moins une matière plastique est choisie parmi gélatine, collagène réticulé et albumine réticulée.

10. Implant médical selon l'une des revendications précédentes, **caractérisé en ce que** le corps creux (1, 21, 51) est expansible et/ou rétractable vis-à-vis de l'atmosphère ambiante par modification de la pression dans son espace intérieur, et/ou
le corps creux (51) présente une branche principale (64) et plusieurs ramifications (66) sortant latéralement de la branche principale (64), dans lequel l'espace intérieur du corps creux (51) s'étend dans la branche principale (64) et dans les ramifications (66) et la conduite d'amenée de fluide (52) et la conduite d'évacuation de fluide (53) sont reliées à la branche principale (64), dans lequel de préférence au moins les ramifications (66) ou des parois des ramifications (66) sont constituées de la matière plastique ou conçues avec la matière plastique, et/ou
le corps creux (1, 21, 51) est constitué d'au moins un matériau résorbable et/ou biodégradable.

11. Système de traitement de défaut osseux présentant un implant médical selon l'une des revendications précédentes et le fluide, dans lequel le fluide contient de l'oxygène et est apte à recevoir du dioxyde de carbone.

12. Système de traitement de défaut osseux selon la revendication 11,
**caractérisé en ce que** le fluide est choisi parmi air, oxygène, solution saline saturée en oxygène, solution de Ringer saturée en oxygène, solution de Ringer-lactate saturée en oxygène, solution tampon phosphate saturée en oxygène et perfluorodécaline saturée en oxygène ou un mélange d'au moins deux des gaz ou liquides mentionnés, et/ou
le système de traitement de défaut osseux présente un matériau de substitution osseux, dans lequel de préférence le matériau de substitution osseux est déposé sur la surface extérieure du corps creux ou peut être appliqué sur la surface extérieure du corps creux.

13. Système de traitement de défaut osseux selon la revendication 12, **caractérisé en ce que** le matériau de substitution osseux est choisi parmi un matériau de substitution osseux non biodégradable, un matériau de substitution osseux partiellement dégradable, un matériau de substitution osseux totalement biodégradable et des mélanges de ceux-ci, ou
le matériau de substitution osseux est choisi parmi tissu osseux autologue, tissu osseux allogénique, hydroxyapatite, carbonate-apatite, β-phosphate tricalcique, α-phosphate tricalcique, dihydrate de calcium, brushite, monétite et des mélanges de ceux-ci, ou le matériau de substitution osseux contient des cellules vivantes et/ou est peuplé de cellules vivantes sur sa surface.

14. Procédé permettant l'aération d'une surface d'un implant médical, en particulier d'un implant médical selon l'une des revendications 1 à 10, comportant de préférence un système de traitement de défaut osseux selon l'une des revendications 11 à 13, présentant :
A) l'introduction d'un fluide contenant de l'oxygène dans un espace intérieur d'un corps creux (1, 21, 51) de l'implant médical ; **caractérisé par :**
B) la distribution d'oxygène gazeux en provenance du fluide dans l'environnement du corps creux (1, 21, 51) à travers une matière plastique délimitant l'espace intérieur du corps creux (1, 21, 51) ;
C) la réception de dioxyde de carbone gazeux en provenance de l'environnement du corps creux (1, 21, 51) dans le fluide à travers la matière plastique délimitant l'espace intérieur ;
D) le passage du fluide à travers l'espace intérieur du corps creux (1, 21, 51) et l'évacuation du fluide de l'espace intérieur du corps creux (1, 21, 51), dans lequel le procédé n'est pas mis en oeuvre pour le traitement médical d'un corps humain ou animal.

15. Procédé selon la revendication 14, **caractérisé en ce que**
avant l'étape A), le corps creux (1, 21, 51) est introduit dans un espace creux et un matériau de substitution osseux est appliqué sur la surface de l'implant médical et/ou est inséré dans l'espace creux entre l'implant médical et des parois intérieures délimitant l'espace creux, et/ou
le procédé permettant la propagation extracorporelle de cellules, en particulier de cellules osseuses, est utilisé, dans lequel, avant l'étape A), les cellules sont appliquées sur la surface extérieure du corps creux (1, 21, 51), de préférence sont appliquées conjointement avec une solution nutritive et/ou des substances favorables à la croissance sur la surface extérieure du corps creux (1, 21, 51).
